# EUROPEAN PATENT SPECIFICATION

(11) **EP 1 696 909 B1**
(45) Date of publication and mention of the grant of the patent: **14.11.2007**
(21) Application number: 04787110.8
(22) Date of filing: 08.09.2004
(51) Int. Cl.: A61K 31/428, A61P 3/10

(54) **BENZOTHIAZOLE DERIVATIVES FOR THE TREATMENT OF DIABETES**
BENZOTHIAZOL-DERIVATE ZUR BEHANDLUNG VON DIABETES
DERIVES DE BENZOTHIAZOLE POUR LE TRAITEMENT DU DIABETE

(30) Priority: 12.09.2003 EP 03102740
(43) Date of publication of application: 06.09.2006
(73) Proprietor: Laboratoires Serono SA, 1267 Coinsins, Vaud (CH)
(72) Inventor: GAILLARD, Pascale Domaine de la Prasle, Bat C2 F-74160 Collonge-sous-Salève (FR); GOTTELAND, Jean-Pierre, F-74160 Beaumont (FR); VITTE, Pierre-Alain, F-74380 Cranves- Sales (FR)
(74) Representative: Viering, Hans-Martin
(86) International application number: PCT/EP2004/052090
(87) International publication number: WO 2005/025567

(56) References cited:
- WO-A-01/47920
- WO-A-03/047570
- LEE YONG HEE ET AL: "c-Jun N-terminal kinase (JNK) mediates feedback inhibition of the insulin signaling cascade" JOURNAL OF BIOLOGICAL CHEMISTRY, AMERICAN SOCIETY OF BIOLOGICAL CHEMISTS, BALTIMORE, MD, US, vol. 278, no. 5, 31 January 2003 (2003-01-31), pages 2896-2902, XP002259338 ISSN: 0021-9258

## Description

### Field of the invention

The present invention is related to benzothiazole derivatives and their tautomers for the treatment of metabolic disorders mediated by insulin resistance or hyperglycemia, comprising diabetes type II, inadequate glucose tolerance, insulin resistance, obesity, polycystic ovary syndrome (PCOS).

### Background of the invention

Diabetes mellitus is a serious metabolic disease that is defined by the presence of chemically elevated levels of blood glucose (hyperglycemia). The term diabetes mellitus encompasses several different hyperglycemic states. These states include Type 1 (insulin-dependent diabetes mellitus or IDDM) and Type 2 (non-insulin dependent diabetes mellitus or NIDDM) diabetes. The hyperglycemia present in individuals with Type 1 diabetes is associated with deficient, reduced, or nonexistent levels of insulin that are insufficient to maintain blood glucose levels within the physiological range. Conventionally, Type 1 diabetes is treated by administration of replacement doses of insulin, generally by a parenteral route.

Type 2 diabetes is an increasingly prevalent disease of aging. It is initially characterized by decreased sensitivity to insulin and a compensatory elevation in circulating insulin concentrations, the latter of which is required to maintain normal blood glucose levels.

The prevalence of insulin resistance in glucose intolerant subjects is well known. Reaven et al (American Journal of Medicine, 60, 80 (1976)) used a continuous infusion of glucose and insulin (insulin/glucose clamp technique) and oral glucose tolerance tests to demonstrate that insulin resistance exists in a diverse group of non-obese, non-ketotic subjects. These subjects ranged from borderline glucose tolerant to overt, fasting hyperglycemia. The diabetic groups in these studies included both insulin dependent (IDDM) and non-insulin dependent (NIDDM) subjects.

Coincident with sustained insulin resistance is the more easily determined hyperinsulinemia, which may be measured by accurate determination of circulating plasma insulin concentration in the plasma of subjects. Hyperinsulinemia may be present as a result of insulin resistance, such as is in obese and/or diabetic (NIDDM) subjects and/or glucose intolerant subjects, or in IDDM subjects, as a consequence of over injection of insulin compared with normal physiological release of the hormone by the endocrine pancreas.

The association of hyperinsulinemia and insulin resistance with obesity has been well established by numerous experimental, clinical and epidemiological studies (Stout, *Metabolism*, 34, 7 (1985)).

The association of hyperinsulinemia and insulin resistance with Polycystic Ovary Syndrome (PCOS) is also well acknowledged (Diamanti-Kandarakis et al.; Therapeutic effects of metformin on insulin resistance and hyperandrogenism in polycystic ovary syndrome; European Journal of Endocrinology 138, 269-274 (1998), Andrea Dunaif; Insulin Resistance and the Polycystic Ovary Syndrome : Mechanism and Implications for Pathogenesis; Endocrine Reviews 18(6), 774-800 (1997)).

Type II diabetes mellitus is currently treated with sulfonylureas, biguanides, such as Metformin and thiazolidenediones, such as Troglitazone, Rosiglitazone or Pioglitazone, as oral hypoglycemic agents.

The compounds of the present invention are disclosed in WO 01/47920 (Applied Research Systems ARS NV) in which benzazoles derivatives of formula (A) are described in particular for the treatment of neuronal disorders, autoimmune diseases, cancer and cardiovascular diseases :

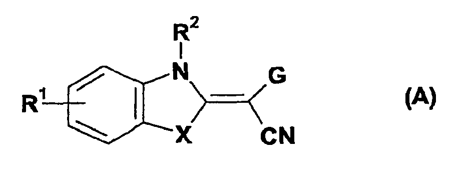

### Summary of the invention

The present invention relates to the use of benzothiazole derivatives of formula (I) for the manufacture of a medicament for the treatment of metabolic disorders mediated by insulin resistance or hyperglycemia, comprising diabetes type II, inadequate glucose tolerance, insulin resistance, obesity, polycystic ovary syndrome (PCOS).

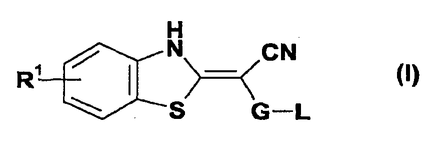

### Detailled description of the invention

The following paragraphs provide definitions of the various chemical moieties that make up the compounds according to the invention and are intended to apply uniformly throughout the specification and claims unless an otherwise expressly set out definition provides a broader definition.

"C₁-C₆-alkyl" refers to alkyl groups having 1 to 6 carbon atoms. This term is exemplified by groups such as methyl, ethyl, n-propyl, isopropyl, n-butyl, isobutyl, *tert*-butyl, n-butyl, n-pentyl, n-hexyl and the like.

"Aryl" refers to an unsaturated aromatic carbocyclic group of from 6 to 14 carbon atoms having a single ring (*e.g*., phenyl) or multiple condensed rings (*e.g*., naphthyl). Preferred aryl include phenyl, naphthyl, phenantrenyl and the like.

"C₁-C₆-alkyl aryl" refers to C₁-C₆-alkyl groups having an aryl substituent, including benzyl, phenethyl and the like.

"Heteroaryl" refers to a monocyclic heteroaromatic, or a bicyclic or a tricyclic fused-ring heteroaromatic group. Particular examples of heteroaromatic groups include optionally substituted pyridyl, pyrrolyl, furyl, thienyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazoly1,1,3,4-triazinyl, 1,2,3-triazinyl, benzofuryl, [2,3-dihydro]benzofuryl, isobenzofuryl, benzothienyl, benzotriazolyl, isobenzothienyl, indolyl, isoindolyl, 3H-indolyl, benzimidazolyl, imidazo[1,2-a]pyridyl, benzothiazolyl, benzoxazolyl, quinolizinyl, quinazolinyl, pthalazinyl, quinoxalinyl, cinnolinyl, napthyridinyl, pyrido[3,4-b]pyridyl, pyrido[3,2-b]pyridyl, pyrido[4,3-b]pyridyl, quinolyl, isoquinolyl, tetrazolyl, 5,6,7,8-tetrahydroquinolyl, 5,6,7,8-tetrahydroisoquinolyl, purinyl, pteridinyl, carbazolyl, xanthenyl or benzoquinolyl.

"C₁-C₆-alkyl heteroaryl" refers to C₁-C₆-alkyl groups having a heteroaryl substituent, including 2-furylmethyl, 2-thienylmethyl, 2-(1H-indol-3-yl)ethyl and the like.

"C₂-C₆-alkenyl" refers to alkenyl groups preferably having from 2 to 6 carbon atoms and having at least 1 or 2 sites of alkenyl unsaturation. Preferable alkenyl groups include ethenyl (-CH=CH₂), n-2-propenyl (allyl, -CH₂CH=CH₂) and the like.

"C₂-C₆-alkenyl aryl" refers to C₂-C₆-alkenyl groups having an aryl substituent, including 2-phenylvinyl and the like.

"C₂-C₆-alkenyl heteroaryl" refers to C₂-C₆-alkenyl groups having a heteroaryl substituent, including 2-(3-pyridinyl)vinyl and the like.

"C₂-C₆-alkynyl" refers to alkynyl groups preferably having from 2 to 6 carbon atoms and having at least 1-2 sites of alkynyl unsaturation, preferred alkynyl groups include ethynyl (-C≡CH), propargyl (-CH₂C≡CH), and the like.

"C₂-C₆-alkynyl aryl" refers to C₂-C₆-alkynyl groups having an aryl substituent, including phenylethynyl and the like.

"C₂-C₆-alkynyl heteroaryl" refers to C₂-C₆-alkynyl groups having a heteroaryl substituent, including 2-thienylethynyl and the like.

"C₃-C₈-cycloalkyl" refers to a saturated carbocyclic group of from 3 to 8 carbon atoms having a single ring (*e.g.*, cyclohexyl) or multiple condensed rings (*e.g.*, norbornyl). Preferred cycloalkyl include cyclopentyl, cyclohexyl, norbornyl and the like.

"C₁-C₆-alkyl cycloalkyl" refers to C₁-C₆-alkyl groups having a cycloalkyl substituent, including cyclohexylmethyl, cyclopentylpropyl, and the like.

"heterocycloalkyl" refers to a C₃-C₈-cycloalkyl group according to the definition above, in which 1 to 3 carbon atoms are replaced by hetero atoms chosen from the group consisting of O, S, NR, R being defined as hydrogen or C₁-C₆ alkyl. Preferred heterocycloalkyl include pyrrolidine, piperidine, piperazine, 1-methylpiperazine, morpholine, and the like.

"C₁-C₆-alkyl heterocycloalkyl" refers to C₁-C₆-alkyl groups having a heterocycloalkyl substituent, including 2-(1-pyrrolidinyl)ethyl, 4-morpholinylmethyl, (1-methyl-4-piperidinyl)methyl and the like.

"Carboxy" refers to the group -C(O)OH.

"C₁-C₆-alkyl carboxy" refers to C₁-C₆-alkyl groups having a carboxy substituent, including 2-carboxyethyl and the like.

"Acyl" refers to the group -C(O)R where R includes H, "C₁-C₆-alkyl", "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl".

"C₁-C₆-alkyl acyl" refers to C₁-C₆-alkyl groups having an acyl substituent, including 2-acetylethyl and the like.

"Aryl acyl" refers to aryl groups having an acyl substituent, including 2-acetylphenyl and the like.

"Heteroaryl acyl" refers to hetereoaryl groups having an acyl substituent, including 2-acetylpyridyl and the like.

"C₃-C₈-(hetero)cycloalkyl acyl" refers to 3 to 8 membered cycloalkyl or heterocycloalkyl groups having an acyl substituent.

"Acyloxy" refers to the group -OC(O)R where R includes H, "C₁-C₆-alkyl", "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl".

"C₁-C₆-alkyl acyloxy" refers to C₁-C₆-alkyl groups having an acyloxy substituent, including 2-(acetyloxy)ethyl and the like.

"Alkoxy" refers to the group -O-R where R includes "C₁-C₆-alkyl", "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl".

"C₁-C₆-alkyl alkoxy" refers to C₁-C₆-alkyl groups having an alkoxy substituent, including 2-ethoxyethyl and the like.

"Alkoxycarbonyl" refers to the group -C(O)OR where R includes "C₁-C₆-alkyl", "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl".

"C₁-C₆-alkyl alkoxycarbonyl" refers to C₁-C₆-alkyl groups having an alkoxycarbonyl substituent, including 2-(benzyloxycarbonyl)ethyl and the like.

"Aminocarbonyl" refers to the group -C(O)NRR' where each R, R' includes independently hydrogen, "C₁-C₆-alkyl", "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl".

"C₁-C₆-alkyl aminocarbonyl" refers to C₁-C₆-alkyl groups having an aminocarbonyl substituent, including 2-(dimethylaminocarbonyl)ethyl and the like.

"Acylamino" refers to the group -NRC(O)R' where each R, R' is independently hydrogen, "C₁-C₆-alkyl", "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl".

"C₁-C₆-alkyl acylamino" refers to C₁-C₆-alkyl groups having an acylamino substituent, including 2-(propionylamino)ethyl and the like.

"Ureido" refers to the group -NRC(O)NR'R" where each R, R', R" is independently hydrogen, "C₁-C₆-alkyl", "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl", and where R' and R", together with the nitrogen atom to which they are attached, can optionally form a 3-8-membered heterocycloalkyl ring.

"C₁-C₆-alkyl ureido" refers to C₁-C₆-alkyl groups having an ureido substituent, including 2-(*N*'-methylureido)ethyl and the like.

"Carbamate" refers to the group -NRC(O)OR' where each R, R' is independently hydrogen, "C₁-C₆-alkyl", "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl".

"Amino" refers to the group -NRR' where each R, R' is independently hydrogen, "C₁-C₆-alkyl", "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl", and where R and R', together with the nitrogen atom to which they are attached, can optionally form a 3-8-membered heterocycloalkyl ring.

"C₁-C₆-alkyl amino" refers to C₁-C₆-alkyl groups having an amino substituent, including 2-(1-pyrrolidinyl)ethyl and the like.

"Ammonium" refers to a positively charged group -N⁺RR'R", where each R, R',R" is independently, "C₁-C₆-alkyl", "C₂-C₆-alkenyl", "C₂-C₆-alkynyl", "C₃-C₈-cycloalkyl", "heterocycloalkyl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl", and where R and R', together with the nitrogen atom to which they are attached, can optionally form a 3-8-membered heterocycloalkyl ring.

"C₁-C₆-alkyl ammonium" refers to C₁-C₆-alkyl groups having an ammonium substituent, including 2-(1-pyrrolidinyl)ethyl and the like.

"Halogen" refers to fluoro, chloro, bromo and iodo atoms.

"Sulfonyloxy" refers to a group -OSO₂-R wherein R is selected from H, "C₁-C₆-alkyl", "C₁-C₆-alkyl" substituted with halogens, *e*.*g*., an -OSO₂-CF₃ group, "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl".

"C₁-C₆-alkyl sulfonyloxy" refers to C₁-C₆-alkyl groups having a sulfonyloxy substituent, including 2-(methylsulfonyloxy)ethyl and the like.

"Sulfonyl" refers to group "-SO₂-R" wherein R is selected from H, "aryl", "heteroaryl", "C₁-C₆-alkyl", "C₁-C₆-alkyl" substituted with halogens, *e*.*g*., an -SO₂-CF₃ group, "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl".

"C₁-C₆-alkyl sulfonyl" refers to C₁-C₆-alkyl groups having a sulfonyl substituent, including 2-(methylsulfonyl)ethyl and the like.

"Sulfinyl" refers to a group "-S(O)-R" wherein R is selected from H, "C₁-C₆-alkyl", "C₁-C₆-alkyl" substituted with halogens, *e*.*g*., an -SO-CF₃ group, "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl".

"C₁-C₆-alkyl sulfinyl" refers to C₁-C₆₋alkyl groups having a sulfinyl substituent, including 2-(methylsulfinyl)ethyl and the like.

"Sulfanyl" refers to groups -S-R where R includes H, "C₁-C₆-alkyl", "C₁-C₆-alkyl" substituted with halogens, *e*.*g*., an -SO-CF₃ group, "C₂-C₆-alkenyl", "C₂-C₆-alkynyl", "C₃-C₈-cycloalkyl". "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl". Preferred sulfanyl groups include methylsulfanyl, ethylsulfanyl, and the like.

"C₁-C₆-alkyl sulfanyl" refers to C₁-C₆-alkyl groups having a sulfanyl substituent, including 2-(ethylsulfanyl)ethyl and the like.

"Sulfonylamino" refers to a group -NRSO₂-R' where each R, R' includes independently hydrogen, "C₁-C₆-alkyl", "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl".

"C₁-C₆-alkyl sulfonylamino" refers to C₁-C₆-alkyl groups having a sulfonylamino substituent, including 2-(ethylsulfonylamino)ethyl and the like.

"Aminosulfonyl" refers to a group -SO₂-NRR' where each R, R' includes independently hydrogen, "C₁-C₆-alkyl", "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl".

"C₁-C₆-alkyl aminosulfonyl" refers to C₁-C₆-alkyl groups having an aminosulfonyl substituent, including 2-(cylohexylaminosulfonyl)ethyl and the like.

"Substituted or unsubstituted" : Unless otherwise constrained by the definition of the individual substituent, the above set out groups, like "alkyl", "alkenyl", "alkynyl", "aryl" and "heteroaryl" etc. groups can optionally be substituted with from 1 to 5 substituents selected from the group consisting of "C₁-C₆-alkyl", "C₂-C₆-alkenyl", "C₂-C₆-alkynyl", "cycloalkyl", "heterocycloalkyl", "C₁-C₆-alkyl aryl", "C₁-C₆-alkyl heteroaryl", "C₁-C₆-alkyl cycloalkyl", "C₁-C₆-alkyl heterocycloalkyl", "amino", "ammonium", "acyl", "acyloxy", "acylamino", "aminocarbonyl", "alkoxycarbonyl", "ureido", "carbamate", "aryl", "heteroaryl", "sulfinyl", "sulfonyl", "alkoxy", "sulfanyl", "halogen", "carboxy", trihalomethyl, cyano, hydroxy, mercapto, nitro, and the like. Alternatively said substitution could also comprise situations where neighbouring substituents have undergone ring closure, notably when vicinal functional substituents are involved, thus forming, *e*.*g*., lactams, lactons, cyclic anhydrides, but also acetals, thioacetals, aminals formed by ring closure for instance in an effort to obtain a protective group.

"Pharmaceutically acceptable salts or complexes" refers to salts or complexes of the below-identified compounds of formula (I) that retain the desired biological activity. Examples of such salts include, but are not restricted to acid addition salts formed with inorganic acids (*e.g.* hydrochloric acid, hydrobromic acid, sulfuric acid, phosphoric acid, nitric acid, and the like), and salts formed with organic acids such as acetic acid, oxalic acid, tartaric acid, succinic acid, malic acid, fumaric acid, maleic acid, ascorbic acid, benzoic acid, tannic acid, pamoic acid, alginic acid, polyglutamic acid, naphthalene sulfonic acid, naphthalene disulfonic acid, methanesulfonic acid and poly-galacturonic acid. Said compounds can also be administered as pharmaceutically acceptable quaternary salts known by a person skilled in the art, which specifically include the quarternary ammonium salt of the formula - NR,R',R" ⁺ Z⁻, wherein R, R', R" is independently hydrogen, alkyl, or benzyl, C₁-C₆-alkyl, C₂-C₆-alkenyl, C₂-C₆-alkynyl, C₁-C₆-alkyl aryl, C₁-C₆-alkyl heteroaryl, cycloalkyl, heterocycloalkyl, and Z is a counterion, including chloride, bromide, iodide, -O-alkyl, toluenesulfonate, methylsulfonate, sulfonate, phosphate, or carboxylate (such as benzoate, succinate, acetate, glycolate, maleate, malate, fumarate, citrate, tartrate, ascorbate, cinnamoate, mandeloate, and diphenylacetate).

"Pharmaceutically active derivative" refers to any compound that upon administration to the recipient, is capable of providing directly or indirectly, the activity disclosed herein.

"Enantiomeric excess" (ee) refers to the products that are obtained by an asymmetric synthesis, i.e. a synthesis involving non-racemic starting materials and/or reagents or a synthesis comprising at least one enantioselective step, whereby a surplus of one enantiomer in the order of at least about 52% ee is yielded.

It was now found that benzothiazole derivatives according to formula I are suitable for the treatment of diabetes type II.

The compounds according to the present invention are those of formula I.

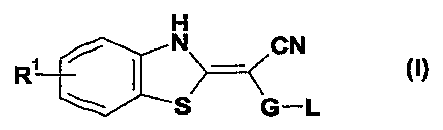

In the compounds according to formula I

G is an unsubstituted or substituted pyrimidinyl group.

L is an unsubstituted or substituted C₁-C₆-alkoxy, or an amino group, or an unsubstituted or a substituted 3-8 membered heterocycloalkyl, containing at least one heteroatom selected from N, O, S (e.g. a piperazine, a piperidine, a morpholine, a pyrrolidine).

R¹ is selected from the group comprising or consisting of hydrogen, sulfonyl, amino, unsubstituted or substituted C₁-C₆-alkyl, unsubstituted or substituted C₂-C₆-alkenyl, unsubstituted or substituted C₂-C₆-alkynyl or C₁-C₆-alkoxy, unsubstituted or substituted aryl (e.g. phenyl), halogen, cyano or hydroxy.

Preferably R¹ is H or C₁-C₃ alkyl (e.g. a methyl or ethyl group).

Formula (I) also comprises its tautomers, its geometrical isomers, its optically active forms as enantiomers, diastereomers and its racemate forms, as well as pharmaceutically acceptable salts thereof. Preferred pharmaceutically acceptable salts of the formula (I) are acid addition salts formed with pharmaceutically acceptable acids like hydrochloride, hydrobromide, sulfate or bisulfate, phosphate or hydrogen phosphate, acetate, benzoate, succinate, fumarate, maleate, lactate, citrate, tartrate, gluconate, methanesulfonate, benzenesulfonate, and *para*-toluenesulfonate salts.

More specifically, the benzothiazole acetonitriles of formula (I) comprise the tautomeric forms, e.g. the below ones :

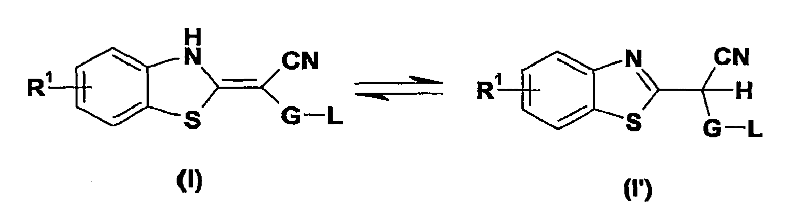

A specific embodiment of the present invention consists in benzothiazole acetonitriles of formula (Ia) in its tautomeric forms, e.g. the below ones :

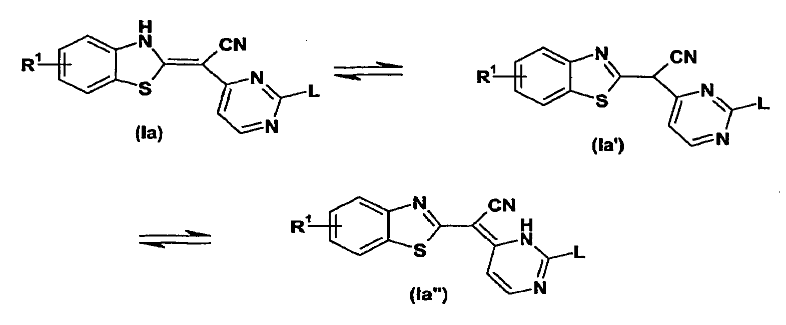

R¹ and L are as defined for formula (I).

According to a specific embodiment, the moiety L is an amino group of the formula -NR³R⁴ wherein R³ and R⁴ are each independently from each other H, unsubstituted or substituted C₁-C₆-alkyl, unsubstituted or substituted C₂-C₆-alkenyl, unsubstituted or substituted C₂-C₆-alkynyl, unsubstituted or substituted C₁-C₆-alkoxy, unsubstituted or substituted aryl, unsubstituted or substituted heteroaryl, unsubstituted or substituted saturated or unsaturated 3-8-membered cycloalkyl, unsubstituted or substituted 3-8-membered heterocycloalkyl, (wherein said cycloalkyl, heterocycloalkyl, aryl or heteroaryl groups may be fused with 1-2 further cycloalkyl, heterocycloalkyl, aryl or heteroaryl group), unsubstituted or substituted C₁-C₆-alkyl aryl, unsubstituted or substituted C₁-C₆-alkyl heteroaryl, unsubstituted or substituted C₁-C₆-alkenyl aryl, unsubstituted or substituted C₁-C₆-alkenyl heteroaryl, unsubstituted or substituted C₁-C₆-alkynyl aryl, unsubstituted or substituted C₁-C₆-alkynyl heteroaryl, unsubstituted or substituted C₁-C₆-alkyl cycloalkyl, unsubstituted or substituted C₁-C₆-alkyl heterocycloalkyl, unsubstituted or substituted C₁-C₆-alkenyl cycloalkyl, unsubstituted or substituted C₁-C₆-alkenyl heterocycloalkyl, unsubstituted or substituted C₁-C₆-alkynyl cycloalkyl, unsubstituted or substituted C₁-C₆-alkynyl heterocycloalkyl.

Alternatively, R³ and R⁴ may form a ring together with the nitrogen to which they are bound.

In a specific embodiment, R³ is hydrogen or a methyl or ethyl or propyl group and R⁴ is selected from the group consisting of unsubstituted or substituted (C₁-C₆)-alkyl, unsubstituted or substituted C₁-C₆ alkyl-aryl, unsubstituted or substituted C₁-C₆-alkylheteroaryl, unsubstituted or substituted cycloalkyl, unsubstituted or substituted heterocycloalkyl, unsubstituted or substituted aryl or heteroaryl and unsubstituted or substituted 4-8 membered saturated or unsaturated cycloalkyl.

In a further specific embodiment, R³ and R⁴ form a substituted or unsubstituted piperazine or a piperidine or a morpholine or a pyrrolidine ring together with the nitrogen to which they are bound, whereby said optional substituent is selected from the group consisting of unsubstituted or substituted C₁-C₆-alkyl, unsubstituted or substituted C₂-C₆-alkenyl, unsubstituted or substituted C₂-C₆-alkynyl, unsubstituted or substituted C₁-C₆-alkoxy, unsubstituted or substituted aryl, unsubstituted or substituted heteroaryl, unsubstituted or substituted saturated or unsaturated 3-8-membered cycloalkyl, unsubstituted or substituted 3-8-membered heterocycloalkyl, (wherein said cycloalkyl, heterocycloalkyl, aryl or heteroaryl groups may be fused with 1-2 further cycloalkyl, heterocycloalkyl, aryl or heteroaryl group), unsubstituted or substituted C₁-C₆-alkyl aryl, unsubstituted or substituted C₁-C₆-alkyl heteroaryl, unsubstituted or substituted C₁-C₆-alkenyl aryl, unsubstituted or substituted C₁-C₆-alkenyl heteroaryl, unsubstituted or substituted C₁-C₆-alkynyl aryl, unsubstituted or substituted C₁-C₆-alkynyl heteroaryl, unsubstituted or substituted C₁-C₆-alkyl cycloalkyl, unsubstituted or substituted C₁-C₆-alkyl heterocycloalkyl, unsubstituted or substituted C₁-C₆-alkenyl cycloalkyl, unsubstituted or substituted C₁-C₆-alkenyl heterocycloalkyl, unsubstituted or substituted C₁-C₆-alkynyl cycloalkyl, unsubstituted or substituted C₁-C₆-alkynyl heterocycloalkyl.

In a specific embodiment L is selected from : 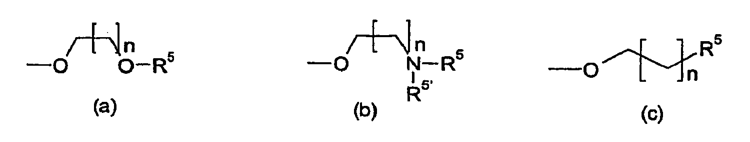 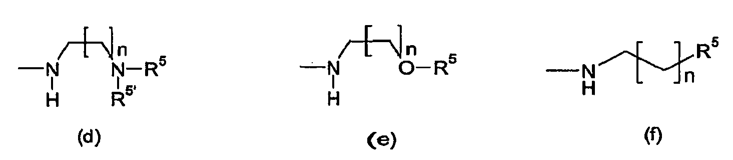 wherein n is 1 to 3, preferably 1 or 2.

R⁵ and R^{5'} are independently selected from each other from the group consisting ofH, substituted or unsubstituted C₁-C₆ alkyl, substituted or unsubstituted aryl or substituted or unsubstituted heteroaryl, substituted or unsubstituted C₁-C₆ alkyl-aryl and substituted or unsubstituted C₁-C₆-alkyl-heteroaryl.

L being the moiety (d) is particularly preferred.

Compounds of formula (I) are suitable for the use as medicament, in particular for the treatment and/or prevention of metabolic disorders mediated by insulin resistance or hyperglycemia, comprising diabetes type II, inadequate glucose tolerance, insulin resistance, obesity, polycystic ovary syndrome (PCOS), in particular for diabetes type II.

Specific examples of compounds of formula I include the following:
1,3-benzothiazol-2-yl(2,6-dimethoxy-4-pyrimidinyl)acetonitrile
1,3 -benzothiazol-2-yl(2- {[2-(1H-imidazol-5-yl)ethyl]amino}-4-pyrimidinyl)acetonitrile
1,3 -benzothiazol-2-yl[2-(1-piperazinyl)-4-pyrimidinyl]acetonitrile
1,3 -benzothiazol-2-yl[2-(4-benzyl-1-piperidinyl)-4-pyrimidinyl]acetonitrile
1,3-benzothiazol-2-yl[2-(4-methyl-1-piperazinyl)-4-pyrimidinyl]acetonitrile
1,3-benzothiazol-2-yl[2-(4-morpholinyl)-4-pyrimidinyl]acetonitrile
1,3-benzothiazol-2-yl(2-(methylamino)-4-pyrimidinyl]acetonitrile
1,3-benzothiazol-2-yl(2-{4-[2-(4-morpholinyl)ethyl]-1-piperazinyl}-4-pyrimidinyl)-acetonitrile
1,3-benzothiazol-2-yl {2-[4-(benzyloxy)-1-piperidinyl]-4-pyrimidinyl}acetonitrile
1,3-benzothiazol-2-yl[2-(4-hydroxy-1-piperidinyl)-4-pyrimidinyl]acetonitrile
1,3-benzothiazol-2-yl(2-{[2-(dimethylamino)ethyl]amino}-4-pyrimidinyl)acetonitrile
1,3-benzothiazol-2-yl[2-(dimethylamino)-4-pyrimidinyl]acetonitrile
1,3-benzothiazol-2-yl{2-[(2-methoxyethyl)amino]-4-pyrimidinyl}acetonitrile
1,3-benzothiazol-2-yl{2-[(2-hydroxyethyl)amino]-4-pyrimidinyl}acetonitrile
1,3-benzothiazol-2-yl[2-(propylamino)-4-pyrimidinyl]acetonitrile
1,3-benzothiazol-2-yl(2-{[3-(1H-imidazol-1-yl)propyl]amino}-4-pyrimidinyl)acetonitrile
1,3 -benzothiazol-2- yl[2-(1-pyrrolidinyl)-4-pyrimidinyl]acetonitrile
1,3-benzothiazol-2-yl{2-[(2-phenylethyl)amino]-4-pyrimidinyl}acetonitrile
1,3-benzothiazol-2-yl(2-{[2-(2-pyridinyl)ethyl]amino}-4-pyrimidinyl)acetonitrile
1,3-benzothiazol-2-yl{2-[(2-pyridinylmethyl)amino]-4pyrimidinyl}acetonitrile
1,3-benzothiazol-2-yl{2-[4-(1H-1,2,3-benzotriazol-1-yl)-1-piperidinyl]-4-pyrimidinyl}acetonitrile
1,3-benzothiazol-2-yl{2-[4-(2-pyrazinyl)-1-piperazinyl]-4-pyrimidinyl}acetonitrile
1,3-benzothiazol-2-yl{2-[4-(2-pyrimidinyl)-1-piperazinyl]-4-pyrimidinyl}acetonitrile
1,3-benzothiazol-2-yl(2-{[2-(3-pyridinyl)ethyl]amino}-4-pyrimidinyl)acetonitrile
1,3-benzothiazol-2-yl(5-bromo-2-{[2-(dimethylamino)ethyl]amino}-4-pyrimidinyl)-acetonitrile
1,3-benzothiazol-2-yl{2-[(2-morpholin-4-ylethyl)amino]pyrimidin-4-yl}acetonitrile
1,3-benzothiazol-2-yl[2-(4-{3-[(trifluoromethyl)sulfonyl]anilino}piperidin-1-yl)pyrimidin-4-yl]acetonitrile
1,3-benzothiazol-2-yl(2-{[3-(2-oxopyrrolidin-1-yl)propyl]amino}pyrimidin-4-yl)-acetonitrile
1,3-benzothiazol-2-yl(2-{methyl[3-(methylamino)propyl]amino}pyrimidin-4-yl)acetonitrile
1,3-benzothiazol-2-yl(2-{[3-(4-methylpiperazin-1-yl)propyl]amino}pyrimidin-4-yl)-acetonitrile
1,3-benzothiazol-2-yl{2-[(3-morpholin-4-ylpropyl)amino]pyrimidin-4-yl}acetonitrile
1,3-benzothiazol-2-yl(2-{[2-(1-methyl-1H-imidazol-4-yl)ethyl]amino}pyrimidin-4-yl)acetonitrile
1,3-benzothiazol-2-yl(2-{[2-(1H-indol-3-yl)ethyl]amino}pyrimidin-4-yl)acetonitrile
1,3 -benzothiazol-2-yl(2-{[2-(4-hydroxyphenyl)ethyl]amino}pyrimidin-4-yl)acetonitrile
tert-butyl ({4-[1,3-benzothiazol-2-yl(cyano)methyl]pyrimidin-2-yl}amino)acetate
{2-[(3-aminopropyl)amino]pyrimidin-4-yl}(1,3-benzothiazol-2-yl)acetonitrile
{2-[(2-aminoethyl)amino]pyrimidin-4-yl}(1,3 -benzothiazol-2-yl)acetonitrile
1,3-benzothiazol-2-yl(2-{[3-(dimethylamino)propyl]amino}pyrimidin-4-yl)acetonitrile
1,3-benzothiazol-2-yl{2-[(2-piperidin-1-ylethyl)amino]pyrimidin-4-yl}acetonitrile
1,3-benzothiazol-2-yl(2-{[2-(1-methyl-1H-imidazol-5-yl)ethyl]amino}pyrimidin-4-yl)acetonitrile
1,3-benzothiazol-2-yl[2-(benzylamino)pyrimidin-4-yl]acetonitrile
isopropyl 3 -({4-[1,3-benzothiazol-2-yl(cyano)methyl]pyrimidin-2-yl}amino)propanoate
1,3 -benzothiazol-2-yl{2-[(3-hydroxypropyl)amino]pyrimidin-4-yl}acetonitrile
1,3-benzothiazol-2-yl{2-[(pyridin-3-ylmethyl)amino]pyrimidin-4-yl}acetonitrile
1,3-benzothiazol-2-yl{2-[(pyridin-4-ylmethyl)amino]pyrimidin-4-yl}acetonitrile
tert-butyl 4-[2-({4-[1,3-benzothiazol-2-yl(cyano)methyl]pyrimidin-2-yl}amino)-ethyl]phenylcarbamate
(2- {[2-(4-aminophenyl)ethyl]amino}pyrimidin-4-yl)(1,3-benzothiazol-2-yl)acetonitrile
1,3-benzothiazol-2-yl(2-{[2-(3,4-dimethoxyphenyl)ethyl]amino}pyrimidin-4-yl)acetonitrile
1,3-benzothiazol-2-yl(2-{[2-(3-methoxyphenyl)ethyl]amino}pyrimidin-4-yl)acetonitrile
1,3-benzothiazol-2-yl(2-{[2-(2-fluorophenyl)ethyl]amino}pyrimidin-4-yl)acetonitrile
1,3-benzothiazol-2-yl[2-({2-[3-(trifluoromethyl)phenyl]ethyl}amino)pyrimidin-4-yl]acetonitrile
1,3 -benzothiazol-2-yl{2-[(2-hydroxy-2-phenylethyl)amino]pyrimidin-4-yl}acetonitrile
1,3-benzothiazol-2-yl{2-[(2-{[3-(trifluoromethyl)pyridin-2-yl]amino}ethyl)amino]-pyrimidin-4-yl}acetonitrile
1,3-benzothiazol-2-yl(2-{[2-(3-chlorophenyl)ethyl]amino}pyrimidin-4-yl)acetonitrile
1,3-benzothiazol-2-yl(2-{[2-(3,4-dichlorophenyl)ethyl]amino}pyrimidin-4-yl)acetonitrile
1,3-benzothiazol-2-yl(2-{[2-(4-methoxyphenyl)emyl]amino}pyrimidin-4-yl)acetonitrile
1,3-benzothiazol-2-yl(2-{[2-(4-methylphenyl)ethyl]amino}pyrimidin-4-yl)acetonitrile
1,3-benzothiazol-2-yl(2-{[2-(3-fluorophenyl)ethyl]amino}pyrimidin-4-yl)acetonitrile
1,3-benzothiazol-2-yl(2-{[2-(4-phenoxyphenyl)ethyl]amino}pyrimidin-4-yl)acetonitrile
1,3-benzothiazol-2-yl(2-{[2-(2-phenoxyphenyl)ethyl]amino}pyrimidin-4-yl)acetonitrile
1,3 -benzothiazol-2-yl(2- {[2-(4-bromophenyl)ethyl]amino}pyrimidin-4-yl)acetonitrile
1,3-benzothiazol-2-yl(2-{[2-(4-fluorophenyl)ethyl]amino}pyrimidin-4-yl)acetonitrile
1,3-benzothiazol-2-yl{2-[(2-[1,1'-biphenyl]-4-ylethyl)amino]pyrimidin-4-yl}acetonitrile
1,3-benzothiazol-2-yl{2-[(2-{4-[hydroxy(oxido)amino]phenyl}ethyl)amino]pyrimidin-4-yl}acetonitrile
1,3-benzothiazol-2-yl(2-{[2-(1H-1,2,4-triazol-1-yl)ethyl]amino}pyrimidin-4-yl)acetonitrile
1,3 -benzothiazol-2-yl(2-{[3-(1H-pyrazol-1-yl)propyl]amino}pyrimidin-4-yl)acetonitrile
4-[2-({4-[1,3-benzothiazol-2-yl(cyano)methyl]pyrimidin-2-yl}amino)ethyl]benzenesulfonamide
{2-[(2-pyridin-3-ylethyl)amino]pyrimidin-4-yl}[5-(trifluoromethyl)-1,3-benzothiazol-2-yl]acetonitrile
1,3-benzothiazol-2-yl{2-[(1H-tetraazol-5-ylmethyl)amino]pyrimidin-4-yl}acetonitrile
1,3-benzothiazol-2-yl[2-(benzyloxy)pyrimidin-4-yl]acetonitrile
1,3-benzothiazol-2-yl{2-[(4-pyridin-3-ylbenzyl)oxy]pyrimidin-4-yl}acetonitrile
1,3 -benzothiazol-2-yl[2-(pyridin-4-ylmethoxy)pyrimidin-4-yl]acetonitrile
1,3-benzothiazol-2-yl[2-(pyridin-2-ylmethoxy)pyrimidin-4-yl]acetonitrile
1,3-benzothiazol-2-yl[2-(3-pyridin-2-ylpropoxy)pyrimidin-4-yl]acetonitrile
1,3-benzothiazol-2-yl{2-[(4-methoxybenzyl)oxy]pyrimidin-4-yl}acetonitrile
1,3-benzothiazol-2-yl[2-(pyridin-3-ylmethoxy)pyrimidin-4-yl]acetonitrile
1,3-benzothiazol-2-yl{2-[2-(4-methoxyphenyl)ethoxy]pyrimidin-4-yl}acetonitrile
1,3-benzothiazol-2-yl[2-([1,1'-biphenyl]-3-ylmethoxy)pyrimidin-4-yl]acetonitrile
1,3-benzothiazol-2-yl{2-[(3,4,5-trimethoxybenzyl)oxy]pyrimidin-4-yl}acetonitrile
1,3-benzothiazol-2-yl{2-[(3,4-dichlorobenzyl)oxy]pyrimidin-4-yl}acetonitrile
1,3-benzothiazol-2-yl[2-({3-[(dimethylamino)methyl]benzyl}oxy)pyrimidin-4-yl]acetonitrile
1,3-benzothiazol-2-yl{2-[(1-oxidopyridin-3-yl)methoxy]pyrimidin-4-yl}acetonitrile
1,3-benzothiazol-2-yl(2-{[4-(morpholin-4-ylmethyl)benzyl]oxy}pyrimidin-4-yl)acetonitrile
1,3-benzothiazol-2-yl{2-[(4-pyridin-2-ylbenzyl)oxy]pyrimidin-4-yl}acetonitrile
1,3-benzotliazol-2-yl(2-{[4-(piperidin-1-ylmethyl)benzyl]oxy}pyrimidin-4-yl)acetonitrile
1,3-benzothiazol-2-yl[2-(4-methoxyphenoxy)pyrimidin-4-yl]acetonitrile
1,3-benzothiazol-2-yl[2-(4-butoxyphenoxy)pyrimidin-4-yl]acetonitrile
{2-[4-(4-acetylpiperazin-1-yl)phenoxy]pyrimidin-4-yl}(1,3-benzothiazol-2-yl)acetonitrile
[2-(4-methoxyphenoxy)pyrimidin-4-yl][5-(trifluoromethyl)-1,3-benzothiazol-2-yl]acetonitrile
N-[2-({4-[1,3-benzothiazol-2-yl(cyano)methyl]pyrimidin-2-yl}amino)ethyl]-4-chlorobenzamide
1,3-benzothiazol-2-yl(2-methoxy-4-pyrimidinyl)acetonitrile
1,3 -benzothiazol-2-yl[2-({4-[(4-methylpiperazin-1-yl)methyl]benzyl}oxy)pyrimidin-4-yl]acetonitrile
1,3-benzothiazol-2-yl[2-({4-[(4-benzyl-piperazin-1-yl)methyl]-benzyl}oxy)pyrimidin-4-yl]acetonitrile
1,3-benzothiazol-2-yl(2-{[4-(piperazin-1-ylmethyl)benzyl]oxy}pyrimidin-4-yl)acetonitrile
1,3-benzothiazol-2-yl[2-({4-[(4-formylpiperazin-1-yl)methyl]benzyl}oxy)pyrimidin-4-yl]acetonitrile
[2-({4-[(4-acetylpiperazin-1-yl)methyl]benzyl}oxy)pyrimidin-4-yl](1,3-benzothiazol-2-yl)acetonitrile
(3H-Benzothiazol-2-ylidene)-{2-[4-(4-[1,2,4]oxadiazol-3-ylmethyl-piperazin-1-ylmethyl)-benzyloxy]-pyrimidin-4-yl}-acetonitrile
4-(4-{4-[(3H-Benzothiazol-2-ylidene)-cyano-methyl]-pyrimidin-2-yloxymethyl}-benzyl)-piperazine-1-carboxylic acid methyl ester
2-[4-(4-{4-[(3H-Benzothiazol-2-ylidene)-cyano-methyl]-pyrimidin-2-yloxymethyl}-benzyl)-piperazin-1-yl]-acetamide
(2-{4-[4-(2-Amino-acetyl)-piperazin-1-ylmethyl]-benzyloxy}-pyrimidin-4-yl)-(3H-benzothiazol-2-ylidene)-acetonitrile
[4-(4- {4-[(3H-Benzothiazol-2-ylidene)-cyano-methyl]-pyrimidin-2-yloxymethyl}-benzyl)-piperazin-1-yl]-acetic acid methyl ester
(3H-Benzothiazol-2-ylidene)-(2-{4-[4-(2-methoxy-ethyl)-piperazin-1-ylmethyl]-benzyloxy}-pyrimidin-4-yl)-acetonitrile
4-(4-{4-[(3H-Benzothiazol-2-ylidene)-cyano-methyl]-pyrimidin-2-yloxymethyl}-benzyl)-piperazine-1-carboxylic acid dimethylamide
(3H-Benzothiazol-2-ylidene)-{2-[4-(4-ethyl-piperazin-1-ylmethyl)-benzyloxy]-pyrimidin-4-yl}-acetonitrile
(3H-Benzolhiazol-2-ylidene)-(2-{4-[4-(2-hydroxy-ethyl)-piperazin-1-ylmethyl]-benzyloxy}-pyrimidin-4-yl)-acetonitrile

The compounds of formula (I) may be obtained according to the methods described in WO 01/47920.

A further aspect of the present invention is related to a pharmaceutical composition composition a comprising a benzothiazole derivative according to formula (I) and at least one additional drug (in particular an anti-diabetes agent). In one embodiment the further diabetes agents are selected from the group comprising or consisting of insulin (or insulin mimicks), aldose reductase inhibitors, alpha-glucosidase inhibitors, sulfonyl urea agents, biguanides (e.g. metformin), thiazolidines (e.g. pioglitizone, rosiglitazone, cf. WO 02/100396), a PTP1B inhibitor, a PPARs agonist or a GSK-3 inhibitor.

Insulins useful with the method of the present invention include rapid acting insulins, intermediate acting insulins, long acting insulins and combination of intermediate and rapid acting insulins.

Aldose reductase inhibitors useful in the method of this invention include those known in the art. These include the non-limiting list of:
a) the spiro-isoquinoline-pyrrolidine tetrone compounds disclosed in U.S. Patent No. 4,927,831 (Malamas), the contents of which are incorporated herein by reference, which includes ARI-509, also known as minalrestat or Spiro[isoquinoline-4(1H), 3'-pyrrolidine]-1,2',3,5'(2H)-tetrone, and analogs thereof,
b) 2- [(4-bromo-2-fluorophenyl)methyl]-6-fluoro- (9CI);
c) the compounds of U.S. Patent No. 4,439,617, the contents of which are incorporated herein by reference, which includes Tolrestat, also known as Glycine, N-[[6-methoxy-5-(trifluoromethyl)-1-naphtalenyl]thioxomethyl]-N-methyl-(9CI) or AY-27773 and analogs thereof;
d) Sorbinil (Registra No. 68367-52-2) also known as Spiro[4H-1-benzopyran-4,4'-imidazoline]-2',5'-dione, 6-fluoro-2,3-dihydro-, (4S)-(9CI) or CP 45634;
e) Methosorbinil;
f) Zopolrestat, which is 1-Phtalazineacetic acid, 3,44-dihydro-4-oxo-3-[[5-(trifluoromethyl)-2-benzothiazolyl]methyl]-(9CI) (Registry No.110703-94-1);
g) Epalrestat, which is 3-Thiazolidineacetic acid, 5-[(2E)-2-methyl-3-phenyl-2-propenylidene]-4-oxo-2-thioxo-, (5Z)-(9CI) (Registry No. 82150-09-9);
h) Zenarestat (Registry No. 112733-40-6) or 3-[(4-bromo-2-fluorophenyl)-methyl]-7-chloro-3,4-dihydro-2,4-dioxo-1(2H)-quinazoline acetic acid;
i) Imirestat, also known as 2,7-difluorospiro(9H-fluorene-9,4'-imidazolidine)-2',5'-dione;
j) Ponalrestat (Registry No.72702-95-5), which is 1-Phtalazineacetic acid, 3-[(4-bromo-2-fluorophenyl)methyl]3,4-dihydro-4-oxo-(9CI) and also known as Stalil or Statyl;
k) ONO-2235, which is 3-Thiazolidineacetic acid, 5-[(2E)-2-methyl-3-phenyl-2-propenylidene-4-oxo-2-thioxo-, (5Z)-(9CI);
l) GP-1447, which is {3-[(4,5,7-trifluorobenzothiazol-2-yl)methyl]-5-methylphenylacetic acid};
m) CT-112, which is 5-(3-ethoxy-4-pentyloxyphenyl)-2,4-thiazolidinedione;
n) BAL-ARI 8, which is Glycine, N[(7-fluoro-9-oxo-9H-xanthen-2-yl)sulfonyl]-N-methyl-)9CI), Reg.No.124066-40-6));
o) AD-5467, which is 2,3-dihydro-2,8-bis(1-methylethyl)-3-thioxox-4H-1,4-benzoxazine-4-acetic acid of the chloride salt form (4H-1,4-Benzoxazine-4-acetic acid, 2,3-dihydro-2,8-bis(1-methylethyl)-3-thioxo-(9CI);
p) ZD5522, which is (3',5'-dimethyl-4'-nitromethylsulfonyl-2-(2-tolyl)acetanilide);
q) 3,4-dihydro-2,8-diisopropyl-3-thioxo-2H-1,4-benzoxazine-4-acetic acid;
r) 1-[(3-bromo-2-benzofuranyl)sulfonyl]-2,4-imidazolidinedione (M-16209),
s) NZ-314, which is 1-Imidazolidineacetic acid, 3-[(3-nitrophenyl)methyl]-2,4,5-trioxo-9(CI) (Registry No.128043-99-2),
t) 1-phtalazineacetic acid, 3,4-dihydro-4-oxo-3-[(5-trifluoromethyl)-2-benzothiazolyl]-methyl];
u) M-79175, which is Spiro[4H-1-benzopyran-4,4'-imidazolidine]-2',5'-dione; 6-fluoro-2,3-dihydro-2-methyl-, (2R, 4S)-(9CI);
v) SPR-210, which is 2H-1,4-Benzothiazine-2-acetic acid, 3,4-dihydro-3-oxo-4-[(4,5,7-trifluoro-2-benzothiazolyl)methyl]-(9CI);
w) Spiro[pyrrolidine-3,6'(5'H)-pyrrolo[1,2,3-de][1,4]benzoxazine]-2,5,5'-trione, 8'-chloro-2'-3'-dihydro-(9CI)(also known as AND 138 or 8-chloro-2',3'-dihydrospiro[pyrolizine-3,6'(5H)-pyrrolo-[1,2,3-de]-[1,4]benzoxazine]2,5,5'-trione);
x) 6-fluoro-2,3-dihydro-2',5'-dioxo-(2S-cis)-spiro[4H-1-benzopyran-4,4'-imidazolidine]-2-carboxamide (also known as SNK-860);

Among the more preferred aldose reductase inhibitors of this invention are minalrestat, Tolrestat, Sorbinil, Methosorbinil, Zopolrestat, Epalrestat, Zenarestat, Imirestat and Ponalrestat or the pharmaceutically acceptable salt forms thereof.

The alpha-glucosidase inhibitors useful for the method of the present invention include miglitol or acarbose, or the pharmaceutically acceptable salt form thereof.

Sulfonylurea agents useful with the method of the present invention include glipizide, Glyburide (Glibenclamide), Clorpropamide, Tolbutamide, Tolazamide and Glimepiride, or the pharmaceutically acceptable salt forms thereof.

Preferably, said supplementary pharmaceutically active agent is selected from the group consisting of a rapid acting insulin, an intermediate acting insulin, a long acting insulin, a combination of intermediate and rapid acting insulins, Inalrestat, Tolrestat, Sorbinil, Methosorbinil, Zopolrestat, Epalrestat, Zenarestat, Imirestat, Ponalrestat, ONO-2235, GP-1447, CT-112, BAL-ARI 8, AD-5467, ZD5522, M-16209, NZ-314, M-79175, SPR-210, ADN 138, or SNK-860, Miglitol, Acarbose, Glipizide, Glyburide, Chlorpropamide, Tolbutamide, Tolazamide, or Glimepriride.

The compounds of the invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous use). Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed.

When employed as pharmaceuticals, the benzothiazole derivatives of this invention are typically administered in the form of a pharmaceutical composition. Such compositions can be prepared in a manner well known in the pharmaceutical art and comprise at least one active compound. Generally, the compounds of this invention are administered in a pharmaceutically effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

The pharmaceutical compositions of these inventions can be administered by a variety of routes including oral, rectal, transdermal, subcutaneous, intravenous, intramuscular, intrathecal, intraperitoneal and intranasal. Depending on the intended route of delivery, the compounds are preferably formulated as either injectable, topical or oral compositions. The compositions for oral administration may take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient Typical unit dosage forms include prefilled, premeasured ampoules or syringes of the liquid compositions or pills, tablets, capsules or the like in the case of solid compositions. In such compositions, the benzothiazole compound is usually a minor component (from about 0.1 to about 50% by weight or preferably from about 1 to about 40% by weight) with the remainder being various vehicles or carriers and processing aids helpful for forming the desired dosing form.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatine; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable carriers known in the art. As above mentioned, the benzothiazole derivatives of formula I in such compositions is typically a minor component, frequently ranging between 0.05 to 10% by weight with the remainder being the injectable carrier and the like.

The above described components for orally administered or injectable compositions are merely representative. Further materials as well as processing techniques and the like are set out in Part 5 of Remington's Pharmaceutical Sciences, 20th Edition, 2000, Marck Publishing Company, Easton, Pennsylvania, which is incorporated herein be reference.

The compounds of this invention can also be administered in sustained release forms or from sustained release drug delivery systems. A description of representative sustained release materials can also be found in the incorporated materials in *Remington's Pharmaceutical Sciences.*

### Example 1 : Preparation of a pharmaceutical formulation

The following formulation examples illustrate representative pharmaceutical compositions according to the present invention being not restricted thereto.

### Formulation 1 - Tablets

A benzothiazole compound of formula I is admixed as a dry powder with a dry gelatin binder in an approximate 1:2 weight ration. A minor amount of magnesium stearate is added as a lubricant. The mixture is formed into 240-270 mg tablets (80-90 mg of active benzothiazole compound per tablet) in a tablet press.

### Formulation 2 - Capsules

A benzothiazole compound of formula I is admixed as a dry powder with a starch diluent in an approximate 1:1 weight ratio. The mixture is filled into 250 mg capsules (125 mg of active benzothiazole compound per capsule).

### Formulation 3 - Liquid

A benzothiazole compound of formula I (1250 mg), sucrose (1.75 g) and xanthan gum (4 mg) are blended, passed through a No. 10 mesh U.S. sieve, and then mixed with a previously prepared solution of microcrystalline cellulose and sodium carboxymethyl cellulose (11:89, 50 mg) in water. Sodium benzoate (10 mg), flavor, and color are diluted with water and added with stirring. Sufficient water is then added to produce a total volume of 5 mL.

### Formulation 4 - Tablets

A benzothiazole compound of formula I is admixed as a dry powder with a dry gelatin binder in an approximate 1:2 weight ratio. A minor amount of magnesium stearate is added as a lubricant. The mixture is formed into 450-900 mg tablets (150-300 mg of active benzothiazole compound) in a tablet press.

### Formulation 5 - Injection

A benzothiazole compound of formula I is dissolved in a buffered sterile saline injectable aqueous medium to a concentration of approximately 5 mg/ml.

### Example 2 : Biological assay

### In vivo assay : Experimental model of type II diabetes (oral postprandial glycemia in db/db mice)

The following assay aims at determining the anti-diabetic effect of the test compounds of formula (I) in a model of postprandial glycemia in db/db mice, *in vivo.*

The assay was performed as follows :

A total of 18 db/db mice (about 8-9 weeks; obtained from IFFACREDO, l'Arbreste, France) were fasted during 20 hours.

3 groups, each consisting of 6 animals were formed :
- Group 1 : The animals were administered (*per os*) a dose of 10 mg/kg of vehicle.
- Group 2 : The animals were administered (*per os*) a dose of 50 mg/kg of the test compound according to formula (I).
- Group 3 : The animals were administered (*per os*) a dose of 100 mg/kg of the test compound according to formula (I).

After oral administration of the compounds of formula (I) solubilized or suspended in CarboxyMethylCellulose (0.5%), Tween 20 (0.25%) and water as vehicle, the animals had access to commercial food (D04, UAR, Villemoisson/Orge, France) *ad libitum.* The diabetic state of the mice was verified by determining the blood glucose level before drug administration. Blood glucose and serum insulin levels were then determined 4 hrs after drug administration.

The determination of the blood glucose level was performed using a glucometer (Precision Q.I.D., Medisense, Abbot, ref. 212.62.31).

The determination of the Insulin level was performed using an ELISA kit (Crystal CHEM, Ref. INSK R020).

Changes in blood glucose and serum insulin of drug treated mice were expressed as a percentage of control (group 1: vehicle treated mice).

Treatment (*per os*) of the animals with test compounds of formula (I), at a dosage of 50 mg/kg, decreased the blood glucose level induced by food intake by about 20-40% and the blood insulin level by about 20-65% compared to the animals treated by the vehicle (Group 1).

For instance, upon using 1,3-benzothiazol-2-yl(2-{[4-(morpholin-4-ylmethyl)benzyl]oxy}-pyrimidin-4-yl)acetonitrile as test compound (100 mg/kg, p.o.), the decrease of 22% in blood glucose level as well as a decrease of 36% in the insulin level was determined (compared to the animals treated by the vehicle (Group 1)):

For instance, upon using 1,3-benzothiazol-2-yl(2-{[2-(3-pyridinyl)ethyl]amino}-4-pyrimidinyl)acetonitrile as test compound (100 mg/kg, p.o.), the decrease of 38% in blood glucose level as well as a decrease of 64% in the insulin level was determined (compared to the animals treated by the vehicle (Group 1)) :

### Reference List

1. Reaven et al (American Journal of Medicine, 60, 80 (1976);
2. Stout, Metabolism, 34, 7 (1985)
3. Diamanti-Kandarakis et al.; European Journal of Endocrinology 138, 269-274 (1998),
4. Andrea Dunaif; Endocrine Reviews 18(6), 774-800 (1997)).
5. WO 01/47920

## Claims

1. Use of benzothiazole derivatives according to formula I 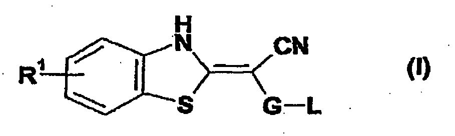 as well as its tautomers, its geometrical isomers, its optically active forms as enantiomers, diastereomers and its racemate forms, as well as pharmaceutically acceptable salts thereof, wherein
G is an pyrimidinyl group.
L is an C₁-C₆-alkoxy, or an amino group, or an 3-8 membered heterocycloalkyl, containing at least one heteroatom selected from N, O, S;
R¹ is selected from the group comprising or consisting of hydrogen, sulfonyl, amino, C₁-C₆-alkyl, C₂-C₆-alkenyl, C₂-C₆-alkynyl or C₁-C₆-alkoxy, aryl, halogen, cyano or hydroxy.
for the preparation of a medicament for the treatment of metabolic disorders mediated by insulin resistance or hyperglycemia, comprising diabetes type II, inadequate glucose tolerance, insulin resistance, obesity, polycystic ovary syndrome (PCOS).

2. Use according to claim 1, wherein the metabolic disorder is diabetes type II.

3. Use according to claim 1 or 2, wherein R¹ is H or C₁-C₃ alkyl.

4. Use according to any of claims 1 to 3, wherein the benzothiazole derivative has any of formulae (Ia), (Ia') or (Ia") : 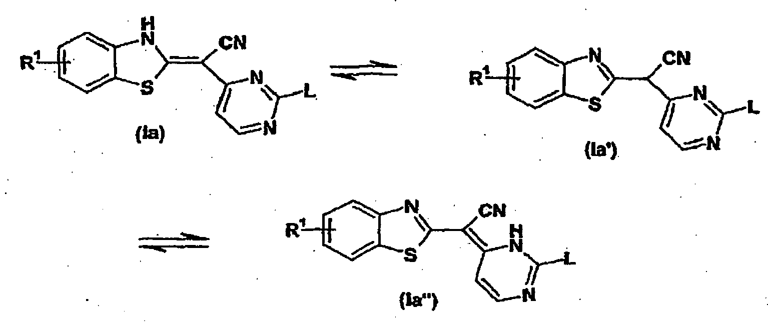 wherein R¹ is is selected from the group consisting of hydrogen, sulfonyl, amino, C₁-C₆-alkyl, C₂-C₆-alkenyl, C₂-C₆-alkynyl or C₁-C₆-alkoxy, aryl, halogen, cyano or hydroxy, and
L is an amino group of the formula -NR³R⁴ wherein R³ and R⁴ are each independently from each other H, C₁-C₆-alkyl, C₂-C₆-alkenyl, C₂-C₆-ahkynyl, C₁-C₆-alkoxy, aryl, heteroaryl, saturated or unsaturated 3-8-membered cycloalkyl, 3-8-membered heterocycloalkyl, (wherein said cycloalkyl heterocycloalkyl, aryl or heteroalyl groups may be fused with 1-2 further cycloalkyl, heterocycloalkyl, aryl or heteroaryl groups), C₁-C₆-alkyl aryl, C₁-C₆-alkyl heteroaryl, C₁-C₆-alkenyl aryl, C₁-C₆-alkenyl heteroaryl, C₁-C₆-alkynyl aryl, C₁-C₆-alkyayl heteroaryl, C₁-C₆-alkyl cycloalkyl, C₁-C₆-alkyl heterocycloalkyl, C₁-C₆-alkenyl cycloalkyl, C₁-C₆-alkenyl heterocycloalkyl, C₁-C₆-alkynyl cycloalkyl, C₁-C₆-alkynyl heterocycloalkyl, or
R³ and R⁴ may form a ring together with the nitrogen to which they are bound.

5. Use according to claim 4, wherein R³ is hydrogen or a methyl or ethyl or propyl group and R⁴ is selected from the group consisting of (C₁-C₆)-alkyl, C₁-C₆ alkyl-aryl, C₁-C₆-alkyl-heteroaryl, cycloalkyl, heterocycloalkyl, aryl or heteroaryl and 4-8 membered saturated or unsaturated cycloalkyl.

6. Use according to claim 4, wherein R³ and R⁴ form an optionally substituted piperazine or a piperidine or a morpholine or a pyrrolidine ring together with the nitrogen to which they are bound, whereby said optional substituent is selected from the group consisting of C₁-C₆-alkyl, C₂-C₆-alkenyl, C₂-C₆-alkynyl, C₁-C₆-alkoxy, aryl, heteroaryl, saturated or unsaturated 3-8-membered cycloalkyl, 3-8-membered heterocycloalkyl (wherein said cycloalkyl, heterocycloalkyl, aryl or heteroaryl groups may be fused with 1-2 further cycloalkyl, heterocycloalkyl, aryl or heteroaryl group), C₁-C₆-alkyl aryl, C₁-C₆-alkyl heteroaryl, C₁-C₆-alkenyl aryl, C₁-C₆-alkenyl heteroaryl, C₁-C₆-alkynyl aryl, C₁-C₆-alkynyl heteroaryl, C₁-C₆-alkyl cycloalkyl, C₁-C₆-alkyl heterocycloalkyl, C₁-C₆-alkenyl cycloalkyl, C₁-C6-alkenyl heterocycloalkyl, C₁-C₆-alkynyl cycloalkyl, C₁-C₆-alkynyl heterocycloalkyl.

7. Use according to any of the preceding claims wherein L is selected from : 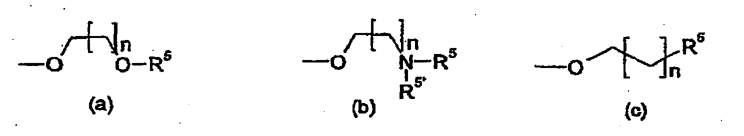 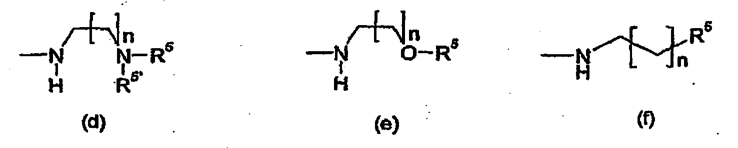 wherein n is 1 to 10, preferably 1 to 6,
R⁵ and R^{5'} are independently selected from each other from the group consisting of H, C₁-C₁₀ alkyl, aryl or hetero-aryl, C₁-C₆ alkyl-aryl and C₁-C₆-alkyl-heteroaryl.

8. Use according to any of the preceding claims wherein the benzothiazole derivative is selected from the following group:
1,3-benzothiazol-2-yl(2,6-dimethoxy-4-pyrimidinyl)acetonitrile
1,3-benzothiazol-2-yl(2-{[2-(1H-imidazol-5-yl)ethyl]amino}-4-pyrimidinyl)acetonitrile
1,3-benzothiazol-2-yl[2-(1-piperazinyl)-4-pyrimidinyl]acetonitrile
1,3-benzothiazol-2-yl[2-(4-benzyl-1-piperidinyl)-4-pyrimidinyl]acetonitrile
1,3-benzothiazol-2-yl[2-(4-methyl-1-piperazinyl)-4-pyrimidinyl]acetonitrile
1,3-benzothiazol-2-yl[2-(4-morpholinyl)-4-pyrimidinyl]acetonitrile
1,3-benzothiazol-2-yl[2-(methylamino)-4-pyrimidinyl]acetonitrile
1,3-benzothiazol-2-yl(2-{4-[2-(4-morpholinyl)ethyl]-1-piperazinyl}-4-pyrimidinyl)-acetonitrile
1,3-benzothiazol-2-yl{2-[4-(benzyloxy)-1-piperidinyl]-4-pyrimidinyl}acetonitrile
1,3-benzothiazol-2-yl[2-(4-hydroxy-1-piperidinyl)-4-pyrimidinyl]acetonitrile
1,3-benzothiazol-2-yl(2-{[2-(dimethylamino)ethyl]amino}-4-pyrimidinyl)acetonitrile
1,3-benzothiazol-2-yl[2-(dimethylamino)-4-pyrimidinyl]acetonitrile
1,3-benzothiazol-2-yl{2-[2-methoxyethyl)amino]-4-pyrimidinyl}acetonitrile
1,3-benzothiazol-2-yl{2-[(2-hydroxyethyl)amino]-4-pyrimidinyl}acetonitrile
1,3-benzothiazol-2-yl[2-(propylamino)-4-pyrimidinyl]acetonitrile
1,3-benzothiazol-2-yl(2-{[3-(1H-imidazol-1-yl)propyl]amino}-4-pyrimidinyl)acetonitrile
1,3-benzothiazol-2-yl[2-(1-pyrrolidinyl)-4-pyrimidinyl]acetonitrile
1,3-benzothiazol-2-yl{2-[(2-phenylethyl)amino]-4-pyrimidinyl}acetonitrile
1,3-benzothiazol-2-yl(2-{[2-(2-pyridinyl)ethyl]amino}-4-pyrimidinyl)acetonitrile
1,3-benzothiazol-2-yl{2-[(2-pyridinylmethyl)amino]-4-pyrimidinyl}acetonitrile
1,3-benzothiazol-2-yl{2-[4-(1H-1,2,3-benzatriazol-1-yl)-1-piperidinyl]-4-pyrimidinyl}acetonitrile
1,3-benzothiazol-2-yl{2-[4-(2-pyrazinyl)-1-piperazinyl]-4-pyrimidinyl}acetonitrile
1,3-benzothiazol-2-yl{2-[4-(2-pyrimidinyl)-1-piperazinyl]-4-pyrimidinyl}acetonitrile
1,3-benzothiazol-2-yl(2-{[2-(3-pyridinyl)ethyl]amino}-4-pyrimidinyl)acetonitrile
1,3-benzothiazol-2-yl(5-bromo-2-{[2-(dimethylamino)ethyl]amino}-4-pyrimidinyl)-acetonitrile
1,3-benzothiazol-2-yl{2-[(2-morpholin-4-ylethyl)amino]pyrimidin-4-yl}acetonitrile
1,3-benzothiazol-2-yl[2-(4-{3-[(trifluoromethyl)sulfonyl]anilino}piperidin-1-yl)pyrimidin-4-yl]acetonitrile
1,3-benzothiazol-2-yl(2-{[3-(2-oxopyrrolidin-1-yl)propyl]amino}pyrimidin-4-yl)-acetonitrile
1,3-benzothiazol-2-yl(2-{methyl[3-(methylamino)propyl]amino}pyrimidin-4-yl)acetonitrile
1,3-benzothiazol-2-yl(2-{[3-(4-methylpiperazin-1-yl)propyl]amino}pyrimidin-4-yl)-acetonitrile
1,3-benzothiazol-2-yl{2-[(3-morpholin-4-ylpropyl)amino]pyrimidin-4-yl}acetonitrile
1,3-benzothiazol-2-yl(2-{[2-(1-methyl1H-imidazol-4-yl)ethyl]amino}pyrimidin-4-yl)acetonitrile
1,3-benzothiazol-2-yl(2-{[2-(1H-indol-3-yl)ethyl)amino}pyrimidin-4-yl)acetonitrile
1,3-benzothiazol-2-yl(2-{[2-(4-hydroxyphenyl)ethyl]amino}pyrimidin-4-yl)acetonitrile
tert-butyl ({4-[1,3-benzothiazol-2-yl(cyano)methyl]pyrimidin-2-yl}amino)acetate
{2-[{3-aminopropyl)amino]pyrimidin-4-yl}(1,3-benzothiazol-2-yl)acetonitrile
{2-[(2-aminoethyl)amino]pyrimidin-4-yl}(1,3-benzothiazol-2-yl)acetonitrile
1,3-benzothiazol-2-yl(2-{[3-(dimethylamino)propyl]amino}pyrimidin-4-yl)acetonitrile
1,3-benzothiazol-2-yl{2-[(2-piperidin-1-ylethyl)amino]pyrimidin-4-yl}acetonitrile
1,3-benzothiazol-2-yl(2-{[2-(1-methyl-1H-imidazol-5-yl)ethyl]amino}pyrimidin-4-yl)acetonitrile
1,3-benzothiazol-2-yl[2-(benzylamino)pyrimidin-4-yl]acetonitrile
isopropyl 3-({4-[1,3-benzothiazol-2-yl(cyano)methyl]pyrimidin-2-yl}amino)propanoate
1,3-benzothiazol-2-yl{2-[(3-hydroxypropyl)amino]pyrimidin-4-yl}acetonitrile
1,3-benzothiazol-2-yl{2-[(pyridin-3-ylmethyl)amino]pyrimidin-4-yl}acetonitrile
1,3-benzothiazol-2-yl{2-[(pyridin-4-ylmethyl)amino]pyrimidin-4-yl}acetonitrile
tert-butyl 4-[2-({4-[1,3-benzothiazol-2-yl(cyano)methyl]pyrimidin-2-yl}amino)-ethyl]phenylcarbamate
(2-{[2-(4-aminophenyl)ethyl]amino]pyrimidin-4-yl)(1,3-benzothiazol-2-yl)acetonitrile
1,3-benzothiazol-2-yl(2-{[2-(3,4-dimethoxyphenyl)ethyl]amino}pyrimidin-4-yl)acetonitrile
1,3-benzothiazol-2-yl(2-{[2-(3-methoxyphenyl)ethyl]amino}pyrimidin-4-yl)acetonitrile
1,3-benzothiazol-2-yl(2-{[2-(2-fluoropheyl)ethyl]amino}pyrimidin-4-yl)acetonitrile
1,3-benzothiazol-2-yl[2-({2-[3-(trifluoromethyl)phenyl]ethyl}amino)pyrimidin-4-yl]acetonitrile
1,3-benzothiazol-2-yl{2-[(2-hydroxy-2-phenylethyl)amino]pyrimidin-4-yl}acetonitrile
1,3-benzothiazol-2-yl{2-[(2-{[3-(trifluoromethyl)pyridin-2-yl]amino}ethyl)amino]-pyrimidin-4-yl}acetonitrile
1,3-benzothiazol-2-yl(2-{[2-(3-chlorophenyl)ethyl]amino}pyrimidin-4-yl)acetonitrile
1,3-benzothiazol-2-yl(2-{[2-(3,4-dichlorophenyl)ethyl]amino}pyrimidin-4-yl)acctonitrile
1,3-benzothiazol-2-yl(2-{[2-(4-methoxyphenyl)ethyl]amino}pyrimidin-4-yl)acetonitrile
1,3-benzothiazol-2-yl(2-{[2-(4-methylphenyl)ethyl]amino}pyrimidin-4-yl)acetonitrile
1,3-benzothiazol-2-yl(2-{[2-(3-fluorophenyl)ethyl]amino}pyrimidin-4-yl)acetonitrile
1,3-benzothiazol-2-yl(2-{[2-(4-phenoxyphenyl)ethyl]amino}pyrimidin-4-yl)acetonitrile
1,3-benzothiazol-2-yl(2-{[2-(2-phenoxyphenyl)ethyl]amino}pyrimidin-4-yl)acetonitrile
1,3-benzothiazol-2-yl(2-{[2-(4-bromophenyl)ethyl]amino}pyrimidin-4-yl)acetanitrile
1,3-benzothiazol-2-yl(2-{[2-(4-fluorophenyl)ethyl]amino}pyrimidin-4-yl)acetonitrile
1,3-benzothiazol-2-yl{2-[(2-1,1'biphenyl]-4-ylethyl)amino]pyrimidin-4-yl}acetonitrile
1,3-benzothiazol-2-yl{2-[(2-{4-[hydroxy(oxido)amino]phenyl}ethyl)amino]pyrimidin-4-yl}acetonitrile
1,3-benzothiazol-2-yl(2-{[2-(1H-1,2,4-triazol-1-yl)ethyl]amino}pyrimidin-4-yl)acetonitrile
1,3-benzothiazol-2-yl(2-{[3-(1H-pyrazol-1-yl)propyl]amino}pyrimidin-4-yl)actonitrile
4-[2-({4-[1,3-benzomiazol-2-yl(cyano)methyl]pyrimidin-2-yl}amino)ethyl]benzenesulfonamide
{2-[(2-pyridin-3-ylethyl)amino]pyrimidin-4-yl}[5-(trifluoromethyl)-1,3-benzothiazol-2-yl]acetonitrile
1,3-benzothiazol-2-yl{2-[(1H-tetraezol-5-ylmethyl)amino]pyrimidin-4-yl}acetonitrile
1,3-benzothiazol-2-yl[2-(benzyloxy)pyrimidin-4-yl]acetonitrile
1,3-benzothiazol-2-yl{2-[(4-pyridin-3-ylbenzyl)oxy]pyrimidin-4-yl}acetonitrile
1,3-benzothiazol-2-yl[2-(pyridin-4-ylmethoxy)pyrimidin-4-yl]acetonitrile
1,3-benzothiazol-2-yl[2-(pyridin-2-ylmethoxy)pyrimidin-4-yl]acetonitrile
1,3-benzothiazol-2-yl[2-(3-pyridin-2-ylpropoxy)pyrimidin-4-yl]acetonitrile
1,3-benzothiazol-2-yl{2-[(4-methoxybenzyl)oxy]pyrimidin-4-yl}acetonitrile
1,3-benzothiazol-2-yl[2-(pyridin-3-ylmethoxy)pyrimidin-4-yl]acetonitrile
1,3-benzothiazol-2-yl{2-[2-(4-methoxyphenyl)ethoxy]pyrimidin-4-yl}acetonile
1,3-benzothiazol-2-yl[2-([1,1'-biphenyl]-3-ylmethoxy)pyrimidin-4-yl]acetonitrile
1,3-benzothiazol-2-yl{2-[(3,4,5-trimethoxybenzyl)oxy]pyrimidin-4-yl}acetonitrile
1,3-benzothiazol-2-yl{2-[(3,4-dichlorobenzyl)oxy]pyrimidin-4-yl}acetonitrile
1,3-benzothiazol-2-yl[2-({3-[(dimethylamino)methyl]benzyl}oxy)pyrimidin-4-yl]acetonitrile
1,3-benzothiazol-2-yl{2-[(1-oxidopyridin-3-yl)methoxy]pyrimidin-4-yl}acetonitrile
1,3-benzothiazol-2-yl(2-{[4-(morpholin-4-ylmethyl)benzyl]oxy}pyrimidin-4-yl)acetonitrile
1,3-benzothiazol-2-yl{2-[(4-pyridin-2-ylbenzyl)oxy]pyrimidin-4-yl}acetonitrile
1,3-benzothiazol-2-yl(2-{[4-(piperidin-1-ylmethyl)benzyl]oxy}pyrimidin-4-yl)acetonitrile
1,3-benzothiazol-2-yl[2-(4-methoxyphenoxy)pyrimidin-4-yl]acetonitrile
1,3-benzothiazol-2-yl[2-(4-butoxyphenoxy)pyrimidin-4-yl]acetonitrile
{2-[4-(4-acetylpiperazin-1-yl)phenoxy]pyrimidin-4-yl}(1,3-benzothiazol-2-yl)acetonitrile
[2-(4-methoxyphenoxy)pyrimidin-4-yl][5-(trifluoromethyl)-1,3-benzothiazol-2-yl]acetonitrile
N-[2-({4-[1,3-benzothiazol-2-yl(cyano)methyl]pyrimidin-2-yl}amino)ethyl]-4-chlorobenzamide
1,3-benzothiazol-2-yl(2-methoxy-4-pyrimidinyl)acetonitrile
1,3-benzothiazol-2-yl[2-({4-[(4-methylpiperazin-1-yl)methyl]benzyl}oxy)pyrimidin-4-yl]acetonitrile
1,3-benzothiazol-2-yl[2-({4-[(4-benzyl-piperazin-1-yl)methyl]-benzyl}oxy)pyrimidin-4-yl]acetonitrile
1,3-benzothiazol-2-yl(2-{[4-(piperazin-1-ylmethyl)benzyl]oxy}pyrimidin-4-yl)acetonitrile
1,3-benzothiazol-2-yl[2-({4-[(4-formylpiperazin-1-yl)methyl]benzyl}oxy)pyrimidin-4-yl]acetonitrile
[2-({4-[(4-acetylpiperazin-1-yl)methyl]benzyl}oxy)pyrimidin-4-yl](1,3-benzothiazol-2-yl)acetonitrile
(3H-Benzothiazol-2-ylidene)-{2-[4-(4-[1,2,4]oxadiazol-3-ylmethyl-piperazin-1-ylmethyl)-benzyloxy]-pyrimidin-4-yl}-acetonitrile
4-(4-{4-[(3H-Benzothiazol-2-ylidene)-cyano-methyl]-pyrimidin-2-yloxymethyl}-benzyl)-piperazine-1-carboxylic acid methyl ester
2-[4-(4-{4-[(3H-Benzothiazol-2-ylidene)-cyano-methyl]-pyrimidin-2-yloxymethyl}-benzyl)-piperazin-1-yl]-acetamide
(2-{4-[4-(2-Amino-acetyl)-piperazin-1-ylmethyl]-benzyloxy}-pyrimidin-4-yl)-(3H-benzothiazol-2-ylidene)-acetonitrile
[4-(4-{4-[(3H-Benzothiazol-2-ylidene)-cyano-methyl]-pyrimidin-2-yloxymethyl}- benzyl)-piperazin-1-yl]-acetic acid methyl ester
(3H-Benzothiazol-2-ylidene)-(2-{4-[4-(2-methoxy-ethyl)-piperazin-1-ylmethyl]-benzyloxy}-pyrimidin-4-yl)-acetonitrile
4-(4-{4-[(3H-Benzothiazol-2-ylidene)-cyano-methyl]-pyrimidin-2-yloxymethyl}- benzyl)-piperazine-1-carboxylic acid dimethylamide
(3H-Benzothiazol-2-ylidene)-{2-[4-(4-ethyl-piperazin-1-ylmethyl)-benzyloxy]-pyrimidin-4-yl}-acetonitrile
(3H-Benzothiazol-2-ylidene)-(2-{4-[4-(2-hydroxy-ethyl)-piperazin-1-ylmethyl]-benzyloxy}-pyrimidin-4-yl)-acetonitrile

9. Use according to any of the preceding claims further comprising at least one supplementary drug selected from the group consisting of insulin, aldose reductase inhibitors, alpha-glucosidase inhibitors, sulfonyl urea agents, biguanides, thiazolidines, PPARs agonists, GSK-3 inhibitors.

10. Use according to claim 9 wherein said supplementary drug is selected from the group consisting of a rapid acting insulin, an intermediate acting insulin, a long acting insulin, a combination of intermediate and rapid acting insulins, Minalrestat, Tolrestat, Sorbinil, Methosorbinil, Zopolrestat, Epalrestat, Zenarestat, Imirestat, Ponalrestat, ONO-2235, GP-1447, CT-112, BAL-ARI 8, AD-5467, ZD5522, M-16209, NZ-314, M-79175, SPR-210, ADN 138, or SNK-860, Miglitol, Acarbose, Glipizide, Glyburide, Chlorpropamide, Tolbutamide, Tolazamide, or Glimepriride.

## Patentansprüche

1. Verwendung von Benzothiazolderivaten der Formel I 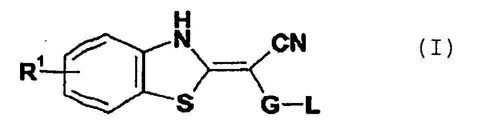 sowie den Tautomeren, den geometrischen Isomeren, den optisch aktiven Formen als Enantiomere, Diastereomere und racemische Formen, sowie pharmazeutisch annehmbare Salze davon, wobei
G eine Pyrimidinylgruppe ist;
L eine C₁-C₆ Alkoxy- oder eine Aminogruppe oder ein 3-8-gliedriger Heterocycloalkylring, der mindestens ein Heteroatom ausgewählt aus N, O und S enthält, ist;
R¹ ausgewählt wird aus der Gruppe umfassend oder bestehend aus Wasserstoff, Sulfonyl, Amino, C₁-C₆ Alkyl, C₂-C₆ Alkenyl, C₂-C₆ Alkinyl oder C₁-C₆ Alkoxy, Aryl, Halogen, Cyano oder Hydroxy
für die Herstellung eines Medikaments für die Behandlung von metabolischen Erkrankungen, die durch Insulinresistenz oder Hyperglykämie vermittelt werden, wobei die Erkrankungen Typ 2 Diabetes, unzureichende Glucosetoleranz, Insulinresistenz, Fettleibigkeit oder polyzystisches Ovarsyndrom (PCOS) umfassen.

2. Verwendung gemäß Anspruch 1, wobei die metabolische Erkrankung Diabetes vom Typ 2 ist.

3. Verwendung gemäß Anspruch 1 oder 2, wobei R¹ H oder C₁-C₃ Alkyl ist.

4. Verwendung gemäß einem der Ansprüche 1 bis 3, wobei das Benzothiazolderivat die Formel (Ia), (Ia') oder (Ia") besitzt: 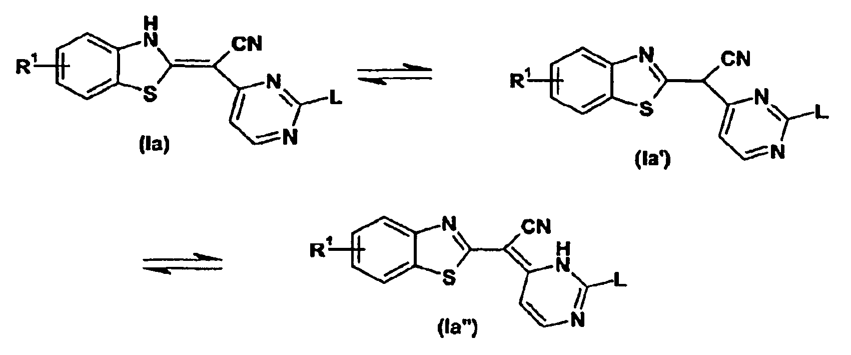 wobei R¹ aus der Gruppe, die aus Wasserstoff, Sulfonyl, Amino, C₁-C₆ Alkyl, C₂-C₆ Alkenyl, C₂-C₆ Alkinyl oder C₁-C₆ Alkoxy, Aryl, Halogen, Cyano oder Hydroxy besteht, ausgewählt wird; und
L eine Aminogruppe der Formel -NR³R⁴- ist, wobei R³ und R⁴ jeweils unabhängig voneinander H, C₁-C₆ Alkyl, C₂-C₆ Alkenyl, C₂-C₆ Alkinyl, C₁-C₆ Alkoxy, Aryl, Heteroaryl, eine gesättigte oder ungesättigte 3 bis 8-gliedrige Cycloalkylgruppe, eine 3 bis 8-gliedrige Heterocycloalkylgruppe (wobei die Cycloalkyl-, Heterocycloalkyl-, Aryl- oder Heteroarylgruppen mit 1 bis 2 weiteren Cycloalkyl-, Heterocycloalkyl-, Aryl- oder Heteroarylgruppen fusioniert sein können), C₁-C₆ Alkylaryl, C₁-C₆ Alkylheteroaryl, C₁-C₆ Alkenylaryl, C₁-C₆ Alkenylheteroaryl, C₁-C₆ Alkinylaryl, C₁-C₆ Alkinylheteroaryl, C₁-C₆ Alkylcycloalkyl, C₁-C₆ Alkylheterocycloalkyl, C₁-C₆ Alkenylcycloalkyl, C₁-C₆ Alkenylheterocycloalkyl, C₁-C₆ Alkinylcycloalkyl, C₁-C₆ Alkinylheterocycloalkyl sind oder
R³ und R⁴ zusammen mit dem Stickstoff, an den sie gebunden sind, einen Ring bilden.

5. Verwendung gemäß Anspruch 4, wobei R³ Wasserstoff oder eine Methyl-, Ethyl- oder Propylgruppe ist und R⁴ ausgewählt wird aus der Gruppe, die aus C₁-C₆ Alkyl, C₁-C₆ Alkylaryl, C₁-C₆ Alkylheteroaryl, Cycloalkyl, Heterocycloalkyl, Aryl oder Heteroaryl und 4 bis 8-gliedrigem gesättigtem oder ungesättigtem Cycloalkyl besteht.

6. Verwendung gemäß Anspruch 4, wobei R³ und R⁴ zusammen mit dem Stickstoff, an den sie gebunden sind, einen gegebenenfalls substituierten Piperazin- oder einen Piperidin- oder einen Morpholin- oder einen Pyrrolidin-Ring bilden, wobei besagter optionaler Substituent aus der Gruppe, die aus C₁-C₆ Alkyl, C₂-C₆ Alkenyl, C₂-C₆ Alkinyl, C₁-C₆ Alkoxy, Aryl, Heteroaryl, gesättigtem oder ungesättigtem 3 bis 8-gliedrigem Cycloalkyl, 3 bis 8-gliedrigem Heterocycloalkyl (wobei die Cycloalkyl-, Heterocycloalkyl-, Aryl- oder Heteroarylgruppen mit 1 bis 2 weiteren Cycloalkyl-, Heterocycloalkyl-, Aryl- oder Heteroarylgruppen fusioniert sein können), C₁-C₆ Alkylaryl, C₁-C₆ Alkylheteroaryl, C₁-C₆ Alkenylaryl, C₁-C₆ Alkenylheteroaryl, C₁-C₆ Alkinylaryl, C₁-C₆ Alkinylheteroaryl, C₁-C₆ Alkylcycloalkyl, C₁-C₆ Alkylheterocycloalkyl, C₁-C₆ Alkenylcycloalkyl, C₁-C₆ Alkenylheterocycloalkyl, C₁-C₆ Alkinylcycloalkyl und C₁-C₆ Alkinylheterocycloalkyl besteht, ausgewählt wird.

7. Verwendung gemäß einem der vorangehenden Ansprüche, wobei L ausgewählt wird aus: 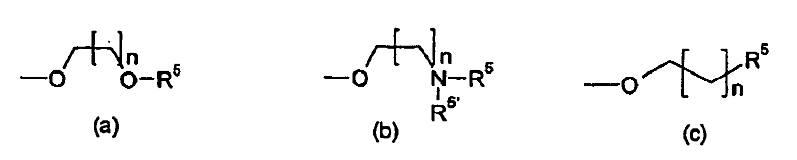 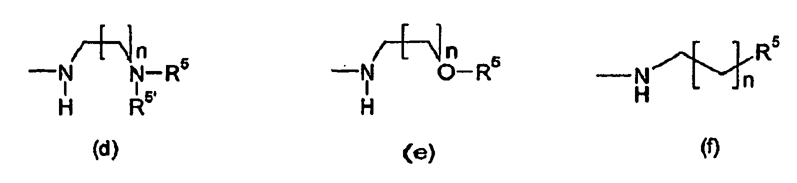 wobei n 1 bis 10, vorzugsweise 1 bis 6 ist,
und R⁵ und R^{5'} unabhängig voneinander aus der Gruppe bestehend aus H, C₁-C₁₀ Alkyl, Aryl oder Heteroaryl, C₁-C₆ Alkylaryl und C₁-C₆ Alkylheteroaryl ausgewählt werden.

8. Verwendung gemäß einem der vorangehenden Ansprüche, wobei das Benzothiazol-Derivat aus der folgenden Gruppe ausgewählt wird:
1,3-benzothiazol-2-yl (2,6-dimethoxy-4-pyrimidinyl) acetonitril
1,3-benzothiazol-2-yl (2-{[2-(1H-imidazol-5-yl)ethyl]amino}-4-pyrimidinyl) acetonitril
1,3-benzothiazol-2-yl [2-(1-piperazinyl)-4-pyrimidinyl] acetonitril
1,3-benzothiazol-2-yl [2-(4-benzyl-1-piperidinyl)-4-pyrimidinyl] acetonitril
1,3-benzothiazol-2-yl [2-(4-methyl-1-piperazinyl)-4-pyrimidinyl] acetonitril
1,3-benzothiazol-2-yl [2-(4-morpholinyl)-4-pyrimidinyl] acetonitril
1,3-benzothiazol-2-yl [2-(methylamino)-4-pyrimidinyl] acetonitril
1,3-benzothiazol-2-yl (2-{4-[2-(4-morpholinyl)ethyl]-1-piperazinyl}-4-pyrimidinyl) acetonitril
1,3-benzothiazol-2-yl {2-[4-(benzyloxy)-1-piperidinyl]-4-pyrimidinyl} acetonitril
1,3-benzothiazol-2-yl [2-(4-hydroxy-1-piperidinyl)-4-pyrimidinyl] acetonitril
1,3-benzothiazol-2-yl (2-{[2-(dimethylamino)ethyl]amino}-4-pyrimidinyl) acetonitril
1,3-benzothiazol-2-yl [2-(dimethylamino)-4-pyrimidinyl] acetonitril
1,3-benzothiazol-2-yl {2-[(2-methoxyethyl)amino]-4-pyrimidinyl} acetonitril
1,3-benzothiazol-2-yl {2-[(2-hydroxyethyl)amino]-4-pyrimidinyl} acetonitril
1,3-benzothiazol-2-yl [2-(propylamino)-4-pyrimidinyl] acetonitril
1,3-benzothiazol-2-yl {2-{[3-(1H-imidazol-1-yl)propyl]amino}-4-pyrimidinyl} acetonitril
1,3-benzothiazol-2-yl [2-(1-pyrrolidinyl)-4-pyrimidinyl] acetonitril
1,3-benzothiazol-2-yl {2-[(2-phenylethyl)amino]-4-pyrimidinyl} acetonitril
1,3-benzothiazol-2-yl (2-{[2-(2-pyridinyl)ethyl]amino}-4-pyrimidinyl} acetonitril
1,3-benzothiazol-2-yl {2-[(2-pyridinylmethyl)amino]-4-pyrimidinyl} acetonitril
1,3-benzothiazol-2-yl {2-[4-(1H-1,2,3-benzotriazol-1-yl)-1-piperidinyl]-4-pyrimidinyl} acetonitril
1,3-benzothiazol-2-yl {2-[4-(2-pyrazinyl)-1-piperazinyl]-4-pyrimidinyl} acetonitril
1,3-benzothiazol-2-yl {2-[4-(2-pyrimidinyl)-1-piperazinyl]-4-pyrimidinyl} acetonitril
1,3-benzothiazol-2-yl (2-[2-(3-pyridinyl)ethyl]amino}-4-pyrimidinyl) acetonitril
1,3-benzothiazol-2-yl (5-bromo-2-{[2-(dimethylamino) ethyl]amino}-4-pyrimidinyl) acetonitril
1,3-benzothiazol-2-yl {2-[(2-morpholin-4-ylethyl)amino]pyridimidin-4-yl}acetonitril
1,3-benzothiazol-2-yl [2-(4-{3-[(trifluoromethyl)sulfonyl] anilino}piperidin-1-yl) pyrimidin-4-yl] acetonitril
1,3-benzothiazol-2-yl (2-{[3-(2-oxopyrrolidin-1-yl)propyl] amino}pyrimidin-4-yl) acetonitril
1,3-benzothiazol-2-yl (2-{methyl[3-(methylamino)propyl] amino}pyrimidin-4-yl) acetonitril
1,3-benzothiazol-2-yl (2-{[3-(4-methylpiperazin-1-yl) propyl]amino} pyrimidin-4-yl) acetonitril
1,3-benzothiazol-2-yl {2-[(3-morpholin-4-ylpropyl)amino] pyrimidin-4-yl} acetonitril
1,3-benzothiazol-2-yl (2-{[2-(1-methyl-1H-imidazol-4-yl) ethyl]amino}pyrimidin-4-yl) acetonitril
1,3-benzothiazol-2-yl (2-{[2-(1H-indol-3-yl)ethyl]amino} pyrimidin-4-yl) acetonitril
1,3-benzothiazol-2-yl (2-{[2-(4-hydroxyphenyl)ethyl]amino} pyrimidin-4-yl) acetonitril
tert-butyl({4-[1,3-benzothiazol-2-yl(cyano)methyl]pyrimidin-2-yl}amino) acetate
{2-[(3-aminopropyl)amino]pyrimidin-4-yl} (1,3-benzothiazol-2-yl) acetonitril
{2-[(2-aminoethyl)amino]pyrimidin-4-yl} (1,3-benzothiazol-2-yl) acetonitril
1,3-benzothiazol-2-yl (2-{[3-(dimethylamino)propyl] amino}pyrimidin-4-yl) acetonitril
1,3-benzothiazol-2-yl {2-[(2-piperidin-1-ylethyl)amino] pyrimidin-4-yl} acetonitril
1,3-benzothiazol-2-yl (2-{[2-(1-methyl-1H-imidazol-5-yl) ethyl] amino)pyrimidin-4-yl) acetonitril
1,3-benzothiazol-2-yl [2-(benzylamino)pyrimidin-4-yl] acetonitril
Isopropyl 3-({4-[1,3-benzothiazol-2-yl(cyano)methyl] pyrimidin-2-yl}amino) propanoat
1,3-benzothiazol-2-yl {2-[(3-hydroxypropyl)amino]pyrimidin-4-yl} acetonitril
1,3-benzothiazol-2-yl {2-[(pyridin-3-ylmethyl)amino] pyrimidin-4-yl} acetonitril
1,3-benzothiazol-2-yl {2-[(pyridin-4-ylmethyl)amino] pyrimidin-4-yl}acetonitril
tert-butyl 4-[2-({4-[1,3-benzothiazol-2-yl(cyano) methyl] pyrimidin-2-yl}amino)ethyl] phenylcarbamat
(2-[2-(4-aminophenyl)ethyl]amino}pyrimidin-4-yl) (1,3-benzothiazol-2-yl) acetonitril
1,3-benzothiazol-2-yl (2-{[2-(3,4-dimethoxyphenyl)ethyl] amino}pyrimidin-4-yl) acetonitril
1,3-benzothiazol-2-yl (2-{[2-(3-methoxyphenyl)ethyl]amino} pyrimidin-4-yl) acetonitril
1,3-benzothiazol-2-yl (2-{[2-(2-fluorophenyl)ethyl]amino} pyrimidin-4-yl) acetonitril
1,3-benzothiazol-2-yl [2-{(2-[3-(trifluoromethyl)phenyl] ethyl}amino)pyrimidin-4-yl] acetonitril
1,3-benzothiazol-2-yl {2-[(2-hydroxy-2-phenylethyl)amino]pyrimidin-4-yl} acetonitril
1,3-benzothiazol-2-yl {2-[(2-{[3-(trifluoromethyl)pyridine-2-yl]amino}ethyl)amino]pyrimidin-4-yl} acetonitril
1,3-benzothiazol-2-yl (2-{[2-(3-chlorophenyl)ethyl]amino} pyrimidin-4-yl) acetonitril
1,3-benzothiazol-2-yl (2-{[2-(3,4-dichlorophenyl)ethyl]amino} pyrimidin-4-yl) acetonitril
1,3-benzothiazol-2-yl (2-{[2-(4-methoxyphenyl)ethyl]amino} pyrimidin-4-yl) acetonitril
1,3-benzothiazol-2-yl (2-{[2-(4-methylphenyl)ethyl]amino} pyrimidin-4-yl) acetonitril
1,3-benzothiazol-2-yl (2-{[2-(3-fluorophenyl)ethyl]amino} pyrimidin-4-yl) acetonitril
1,3-benzothiazol-2-yl (2-{[2-(4-phenoxyphenyl)ethyl]amino} pyrimidin-4-yl) acetonitril
1,3-benzothiazol-2-yl (2-{[2-(2-phenoxyphenyl)ethyl]amino} pyrimidin-4-yl) acetonitril
1,3-benzothiazol-2-yl (2-{[2-(4-bromophenyl)ethyl]amino} pyrimidin-4-yl) acetonitril
1,3-benzothiazol-2-yl (2-{[2-(4-fluorophenyl)ethyl]amino} pyrimidin-4-yl) acetonitril
1,3-benzothiazol-2-yl {2-[(2-[1,1-biphenyl]-4-ylethyl) amino]pyrimidin-4-yl} acetonitril
1,3-benzothiazol-2-yl {2-[(2-{4-[hydroxy(oxido)amino]phenyl} ethyl)amino]pyrimidin-4-yl} acetonitril
1,3-benzothiazol-2-yl (2-{[2-(1H-1,2,4-triazol-1-yl)ethyl] amino}pyrimidin-4- yl) acetonitril
1,3-benzothiazol-2-yl (2-{[3-(1H-pyrazol-1-yl)propyl]amino} pyrimidin-4-yl) acetonitril
4-[2-({4-[1,3-benzothiazol-2-yl(cyano)methyl]pyrimidin-2-yl} amino)ethyl]benzene sulfonamid
{2-[(2-pyridin-3-ylethyl)amino]pyrimidin-4-yl} [5-(trifluoromethyl)-1,3-benzothiazol-2-yl] acetonitril
1,3-benzothiazol-2-yl {2-[(1H-tetrazol-5-ylmethyl)amino] pyrimidin-4-yl} acetonitril
1,3-benzothiazol-2-yl [2-(benzyloxy)pyrimidin-4-yl] acetonitril
1,3-benzothiazol-2-yl {2-[(4-pyridin-3-ylbenzyl)oxy] pyrimidin-4-yl} acetonitril
1,3-benzothiazol-2-yl [2-(pyridin-4-ylmethoxy)pyrimidin-4-yl] acetonitril
1,3-benzothiazol-2-yl[2-(pyridin-2-ylmethoxy)pyrimidin-4-yl] acetonitril
1,3-benzothiazol-2-yl [2-(3-pyridin-2-ylpropoxy)pyrimidin-4-yl] acetonitril
1,3-benzothiazol-2-yl {2-[(4-methoxybenzyl)oxy]pyrimidin-4-yl} acetonitril
1,3-benzothiazol-2-yl [2-(pyridin-3-ylmethoxy)pyrimidin-4-yl] acetonitril
1,3-benzothiazol-2-yl {2-[2-(4-methoxyphenyl) ethoxy] pyrimidin-4-yl} acetonitril
1,3-benzothiazol-2-yl [2-([1,1'-biphenyl]-3-ylmethoxy) pyrimidin-4-yl] acetonitril
1,3-benzothiazol-2-yl {2-[(3,4,5-trimethoxybenzyl)oxy] pyrimidin-4-yl} acetonitril
1,3-benzothiazol-2-yl {2-[(3,4-dichlorobenzyl)oxy] pyrimidin-4-yl} acetonitril
1,3-benzothiazol-2-yl [2-({3-[(dimethylamino)methyl]benzyl} oxy)pyrimidin-4-yl] acetonitril
1,3-benzothiazol-2-yl {2-[(1-oxidopyridin-3-yl)methoxy] pyrimidin-4-yl} acetonitril
1,3-benzothiazol-2-yl (2-{[4-(morpholin-4-ylmethyl)benzyl] oxy}pyrimidin-4-yl) acetonitril
1,3-benzothiazol-2-yl {2-[(4-pyridin-2-ylbenzyl)oxy] pyrimidin-4-yl} acetonitril
1,3-benzothiazol-2-yl (2-{[4-(piperidin-1-ylmethyl)benzyl] oxy}pyrimidin-4-yl) acetonitril
1,3-benzothiazol-2-yl [2-(4-methoxyphenoxy)pyrimidin-4-yl] acetonitrile
1,3-benzothiazol-2-yl [2-(4-butoxyphenoxy)pyrimidin-4-yl] acetonitril
{2[4-(4-acetylpiperazin-1-yl)phenoxy]pyrimidin-4-yl} (1,3-benzothiazol-2-yl) acetonitril
[2-(4-methoxyphenoxy)pyrimidin-4-yl] [5-(trifluoromethyl)-1,3-benzothiazol-2-yl) acetonitril
N-[2-({4-[1,3-benzothiazol-2-yl(cyano)methyl]pyrimidin-2-yl}amino)ethyl]-4-chlorobenzamid
1,3-benzothiazol-2-yl (2-methoxy-4-pyrimidinyl) acetonitril 1,3-benzothiazol-2-yl [2-({4-[(4-methylpiperazin-1-yl)methyl] benzyl}oxy)pyrimidin-4-yl] acetonitril
1,3-benzothiazol-2-yl [2-({4-[(4-benzyl-piperazin-1-yl)methyl] benzyl}oxy)pyrimidin-4-yl] acetonitril
1,3-benzothiazol-2-yl (2-{[4-(piperazin-1-ylmethyl)benzyl] oxy}pyrimidin-4-yl) acetonitril
1,3-benzothiazol-2-yl [2-({4-[(4-formylpiperazin-1-yl) methyl]benzyl}oxy)pyrimidin-4- yl] acetonitril
[2-({4-[(4-acetylpiperazin-l-yl)methyl]benzyl}oxy)pyrimidin-4-yl] (1,3-benzothiazol-2-yl) acetonitril
(3H-Benzothiazol-2-yliden)-{2-[4-(4-[1,2,4]oxadiazol-3-ylmethyl-piperazin-1-ylmethyl)-benzyloxy]-pyrimidin-4-yl} acetonitril
4-(4-{4-[(3H-Benzothiazol-2-yliden)-cyano-methyl]-pyrimidin-2-yloxymethyl}-benzyl)-piperazin-1-carbonsäuremethylester
2-[4-(4-{4-[(3H-Benzothiazol-2-yliden)-cyano-methyl]-pyrimidin-2-yloxymethyl}- benzyl)-piperazin-1-yl]-acetamid
(2-{4-[4-(2-Amino-acetyl)-piperazin-1-ylmethyl]-benzyloxy}-pyrimidin-4-yl)-(3H-benzothiazol-2-yliden) acetonitril
4-(4-{4-[(3H-Benzothiazol-2-yliden)-cyano-methyl]-pyrimidin-2-yloxymethyl}-benzyl)-piperazin-1-yl] Essigsäuremethylester
(3H-Benzothiazol-2-yliden)-(2-{4-[4-(2-methoxyethyl)-piperazin-1-ylmethyl]-benzyloxy}-pyrimidin-4-yl} acetonitril
4-(4-{4-[(3H-Benzothiazol-2-yliden)-cyano-methyl]-pyrimidin-2-yloxymethyl}-benzyl)-piperazin-1-carbonsäuredimethylamid
(3H-Benzothiazol-2-yliden)-{2-[4-(4-ethyl-piperazin-1-ylmethyl)-benzyloxy]-pyrimidin-4-yl} acetonitril
(3H-Benzothiazol-2-yliden)-(2-{4-[4-(2-hydroxy-ethyl)-piperazin-1-ylmethyl]-benzyloxy}-pyrimidin-4-yl) acetonitril

9. Verwendung gemäß einem der vorangehenden Ansprüche, wobei die Verwendung ferner die Verwendung mindestens eines ergänzenden Medikaments, das aus der Gruppe, die aus Insulin, Aldosereduktase-Inhibitoren, alpha-Glucosidase-Inhibitoren, Sulfonylharnstoff Agenzien, Biguaniden, Thiazolidinen, PPARs Agonisten und GSK-3 Inhibitoren besteht, ausgewählt wird, umfasst.

10. Verwendung gemäß Anspruch 9, wobei besagtes ergänzendes Medikament aus der Gruppe, die aus einem schnell wirkenden Insulin, einem mittelfristig wirkenden Insulin, einem lang wirkenden Insulin, einer Kombination von mittelfristig und schnell wirkenden Insulinen, Minalrestat, Tolrestat, Sorbinil, Methosorbinil, Zopolrestat, Epalrestat, Zenarestat, Imirestat, Ponalrestat, ONO-2235, GP-1447, CT-112, BAL-ARI 8, AD-5467, ZD5522, M-16209, NZ-314, M-79175, SPR-210, ADN 138 oder SNK-860, Miglitol, Acarbose, Glipizid, Glyburid, Chlorpropamid, Tolbutamid, Tolazamid oder Glimepririd besteht, ausgewählt wird.

## Revendications

1. Utilisation de dérivés de benzothiazole répondant à la formule I 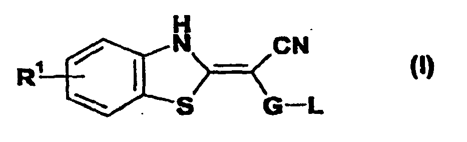 ainsi que leurs tautomères, leurs isomères géométriques, leurs formes optiquement actives telles que énantiomères, diastéréoisomères et leurs formes racémates, ainsi que de leurs sels pharmaceutiquement acceptables, formule dans laquelle
G représente un groupe pyrimidinyle,
L représente un groupe alcoxy en C₁-C₆, ou un groupe amino ou un groupe hétérocycloalkyle à 3 - 8 chaînons contenant au moins un hétéroatome choisi parmi N, O, S,
R¹ est choisi dans l'ensemble comprenant ou englobant l'hydrogène et les groupes sulfonyle, amino, alkyle en C₁-C₆, alcényle en C₂-C₆, alcynyle en C₂-C₆ ou alcoxy en C₁-C₆, aryle, les atomes d'halogène et les groupes cyano ou hydroxy,
pour la préparation d'un médicament pour le traitement de troubles métaboliques favorisés par la résistance à l'insuline ou l'hyperglycémie, comprenant le diabète de type II, la tolérance inadéquate au glucose, la résistance à l'insuline, l'obésité, la polykystose ovarienne (PCOS).

2. Utilisation selon la revendication 1, dans laquelle le trouble métabolique est le diabète de type II.

3. Utilisation selon la revendication 1 ou 2, dans laquelle R¹ est H ou un groupe alkyle en C₁-C₃.

4. Utilisation selon l'une quelconque des revendications 1 à 3, dans laquelle le dérivé de benzothiazole possède n'importe laquelle des formules (Ia), (Ia') et (Ia") : 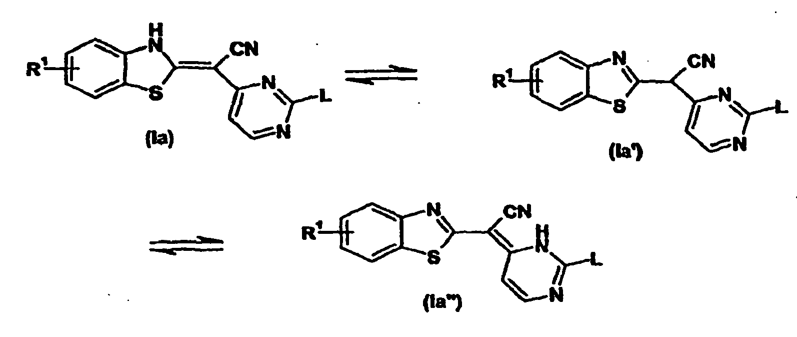 dans lesquelles R¹ est choisi dans l'ensemble comprenant l'atome d'hydrogène, les atomes d'halogène et les groupes sulfonyle, amino, alkyle en C₁-C₆, alcényle en C₂-C₆, alcynyle en C₂-C₆, alcoxy en C₁-C₆, aryle, cyano ou hydroxy, et
L représente un groupe amino de formule -NR³R⁴, dans laquelle R³ et R⁴ représentent chacun indépendamment l'un de l'autre H ou un groupe alkyle en C₁-C₆, alcényle en C₂-C₆, alcynyle en C₂-C₆, alcoxy en C₁-C₆, aryle, hétéroaryle, cycloakyle à 3 - 8 chaînons saturé ou non saturé, hétérocycloalkyle à 3 - 8 chaînons (lesdits groupes cycloalkyle, hétérocycloalkyle, aryle et hétéroaryle pouvant être fusionnés avec 1 - 2 groupes cycloalkyle, hétérocycloalkyle, aryle ou hétéroaryle supplémentaires), alkyle en C₁-C₆-aryle, alkyle en C₁-C₆-hétéroaryle, alcényle en C₁-C₆-aryle, alcényle en C₁-C₆-hétéroaryle, alcynyle en C₁-C₆-aryle, alcynyle en C₁-C₆-hétéroaryle, alkyle en C₁-C₆-cycloalkyle, alkyle en C₁-C₆-hétérocycloalkyle, alcényle en C₁-C₆-cycloalkyle, alcényle en C₁-C₆-hétérocycloalkyle, alcynyle en C₁-C₆-cycloalkyle, alcynyle en C₁-C₆-hétérocycloalkyle, ou
R³ et R⁴ peuvent former un cycle conjointement avec l'atome d'azote auquel ils sont liés.

5. Utilisation selon la revendication 4, dans laquelle R³ représente un atome d'hydrogène ou un groupe méthyle ou éthyle ou propyle et R⁴ est choisi dans l'ensemble comprenant les groupes alkyle en C₁-C₆, alkyle en C₁-C₆-aryle, alkyle en C₁-C₆-hétéroaryle, cycloalkyle, hétérocycloalkyle, aryle ou hétéroaryle et cycloalkyle à 4 - 8 chaînons, saturé ou insaturé.

6. Utilisation selon la revendication 4, dans laquelle R³ et R⁴ forment un cycle pipérazine ou pipéridine ou morpholine ou pyrrolidine éventuellement substitué, conjointement avec l'atome d'azote auquel ils sont liés, et ou ledit substituant optionnel est choisi dans l'ensemble comprenant les groupes alkyle en C₁-C₆, alcényle en C₂-C₆, alcynyle en C₂-C₆, alcoxy en C₁-C₆, aryle, hétéroaryle, cycloakyle à 3 - 8 chaînons saturé ou non saturé, hétérocycloalkyle à 3 - 8 chaînons (lesdits groupes cycloalkyle, hétérocycloalkyle, aryle et hétéroaryle pouvant être fusionnés avec 1 - 2 groupes cycloalkyle, hétérocycloalkyle, aryle ou hétéroaryle supplémentaires), alkyle en C₁-C₆-aryle, alkyle en C₁-C₆-hétéroaryle, alcényle en C₁-C₆-aryle, alcényle en C₁-C₆-hétéroaryle, alcynyle en C₁-C₆-aryle, alcynyle en C₁-C₆-hétéroaryle, alkyle en C₁-C₆-cycloalkyle, alkyle en C₁-C₆-hétérocycloalkyle, alcényle en C₁-C₆-cycloalkyle, alcényle en C₁-C₆-hétérocycloalkyle, alcynyle en C₁-C₆-cycloalkyle, alcynyle en C₁-C₆-hétérocycloalkyle.

7. Utilisation selon l'une quelconque des revendications précédentes, dans laquelle L est choisi parmi : 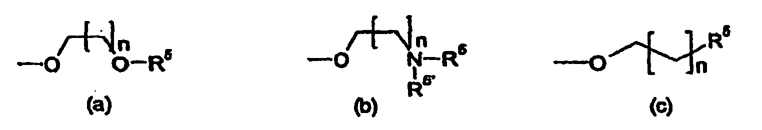 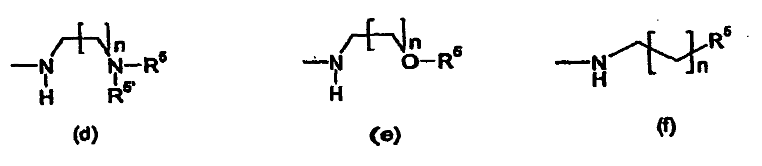 où n vaut de 1 à 10, de préférence de 1 à 6,
R⁵ et R^{5'} sont choisis indépendamment l'un de l'autre dans l'ensemble comprenant H et les groupes alkyle en C₁-C₁₀, aryle ou hétéroaryle, alkyle en C₁-C₆-aryle et alkyle en C₁-C₆-hétéroaryle.

8. Utilisation selon l'une quelconque des revendications précédentes, dans laquelle le dérivé de benzothiazole est choisi dans le groupe suivant :
1,3-benzothiazol-2-yl (2,6 - diméthoxy-4-pyrimidinyl)acétonitrile, 1,3-benzothiazol-2-yl(2-{[2-(1H-imidazol-5-yl)éthyl]amino}-4-pyrimidinyl) -acétonitrile, 1 ,3-benzothiazol-2-yl [2-(1- pipérazinyl) -4-pyrimidinyl]acétonitrile, 1,3-benzothiazol-2-yl[2-(4-benzyl-1-pipéridinyl)-4-pyrimidinyl] acétonitrile , 1,3-benzothiazol- 2-yl[2-(4-méthyl-1-pipérazinyl)-4-pyrimidinyl]acétonitrile, 1,3-benzothiazol-2-yl[2-(4-morpholinyl)-4-pyrimidinyl] acétonitrile, 1,3-benzothiazol-2-yl[2-(méthylamino)-4-pyrimidinyl]acétonitrile, 1,3-benzothiazol-2-yl(2-{4-[2-(4-morpholinyl)éthyl]-1-pipérazinyl}-4-pyrimidinyl)-acétonitrile, 1,3-benzothiazol-2-yl{2-(4-(benzyloxy)-1-pipéridinyl]-4-pyrimidinyl} acétonitrile, 1,3-benzothiazol-2-yl [2-(4-hydroxy-1-pipéridinyl)-4-pyrimidinyl] acétonitrile, 1,3-benzothiazol-2-yl(2-{[2-(diméthyl- amino)éthyl]amino}-4-pyrimidinyl)acétonitrile, 1,3-benzothiazol-2-yl-[2-(diméthylamino)-4-pyrimidinyl]acétonitrile, 1,3-benzothiazol-2- yl- {2-[(2- méthoxyéthyl)amino] -4-pyrimidinyl} acétonitrile, 1,3-benzothiazol-2-yl(2- [(2-hydroxyéthyl) amino]-4-pyrimidinyl}acétonitrile, 1,3-benzothiazol-2-yl[2-(propylamino)-4-pyrimidinyl] acétonitrile, 1,3-benzothiazol-2-yl(2-{[3-(1H-imidazol-1-yl)propyl]amino}-4-pyrimidinyl)acétonitrile, 1,3-benzothiazol-2-yl[2-(1-pyrrolidinyl)-4-pyrimidinyl]acétonitrile, 1,3-benzothiazol-2-yl{2-[(2-phényléthyl) amino]-4-pyrimidinyl} acétonitrile, 1,3-benzothiazol-2-yl(2-{[2-(2-pyridinyl)éthyl]amino}-4-pyrimidinyl)acétonitrile, 1,3-benzothiazol-2-yl{2-[(2-pyridinylméthyl)amino]-4-pyrimidinyl} acétonitrile, 1,3-benzothiazol-2-yl{2-[4-(1H-1,2,3-benzotriazol-1-yl)-1-pipéridinyl]-4-pyrimidinyl} acétonitrile, 1,3-benzothiazol-2-yl{2-[4-(2-pyrazinyl)-1-pipérazinyl]-4-pyrimidinyl} acétonitrile, 1,3-benzothiazol-2-yl{2-[4-(2-pyrimidinyl)-1-pipérazinyl]-4-pyrimidinyl} acétonitrile, 1,3-benzothiazol- 2-yl(2-{[2-(3- pyridinyl)éthyl] amino}-4- pyrimidinyl)- acétonitrile, 1,3-benzothiazol-2-yl(5-bromo-2-{[2-(diméthylamino)- éthyl] amino}-4-pyrimidinyl)acétonitrile, 1,3-benzothiazol-2-yl{2-[(2-morpholin-4-yléthyl)amino]pyrimidin-4-yl}acétonitrile, 1,3-benzothiazol-2-yl[2-(4-{3-[(trifluorométhyl) sulfonyl]anilino} pipéridin-1-yl)pyrimidin-4-yl]acétonitrile, 1,3-benzothiazol-2-yl(2-{[3-(2-oxo-pyrrolidin-1-yl)propyl]amino} pyrimidin-4-yl)-acétonitrile, 1,3-benzothiazol-2-yl(2-{méthyl[3-(méthylamino)propyl]amino} pyrimidin-4-yl)-acétonitrile, 1,3-benzothiazol-2-yl(2-{[3-(4-méthylpipérazin-1-yl)- propyl] amino} pyrimidin-4-yl)acétonitrile, 1,3-benzothiazol-2-yl{2-[(3-morpholin-4-ylpropyl)amino]pyrimidin-4-yl}acétonitrile, 1,3-benzothiazol-2-yl(2-{[2-(1-méthyl-1H-imidazol-4-yl)éthyl]amino}pyrimidin- 4yl)acétonitrile, 1,3-benzothiazol-2-yl(2-{[2-(1H-indol-3-yl)éthyl]-amino}pyrimidin-4-yl)acétonitrile, 1,3-benzothiazol- 2-y(2-{[2-(4-hydroxyphényl)éthyl]amino} pyrimidin-4-yl)acétonitrile, ({4-[1,3-benzothiazol-2-yl(cyano )méthyl]pyrimidin-2-yl}amino)acétate de tert butyle, {2-[(3-aminopropyl)amino]pyrimidin-4-yl}(1,3-benzothiazol-2-yl)acétonitrile, {{2-[(2-aminoéthyl)amino ]pyrimidin-4-yl}(1,3-benzothiazol-2-yl)acétonitrile, 1,3-benzothiazol-2-yl(2-{[3-(diméthyl -amino)propyl]amino}pyrimidin-4-yl)acétonitrile, 1,3-benzothiazol-2-yl{2-[(2-pipéridin-1-yléthyl)amino]pyrimidin-4-yl} acétonitrile, 1,3-benzothiazol -2-yl(2-{[2- (1-méthyl-1H- imidazol-5-yl)éthyl]amino} pyrimidin-4yl)acétonitrile, 1,3-benzothiazol-2-yl[2-(benzylamino)-pyrimidin-4-yl]acétonitrile, 3-({4-[1,3- benzothiazol- 2-yl(cyano)-méthyl]pyrimidin-2-yl}amino )propanoate d'isopropyle, 1,3-benzothiazol- 2-yl{2-[(3-hydroxypropyl) amino] pyrimidin-4-yl} acétonitrile, 1,3-benzothiazol- 2-yl{2-[(pyridin- 3-ylméthyl)amino] pyrimidin-4-yl} acétonitrile, 1,3-benzothiazol-2-yl{2-[(pyridin-4-ylméthyl)amino]pyrimidin-4-yl}acétonitrile, 9-[2-({4-[1,3-benzothiazol -2-yl(cyano)méthyl]pyrymidin- 2-yl}amino)éthyl]phénylcarbamate de tert-butyle, (2-{[2-(4-aminophényl)éthyl]amino}pyrimidin-4-yl)(1,3-benzothiazol-2-yl)acétonitrile, 1,3-benzothiazol-2-yl(2-{[2-(3,4-diméthoxyphényl)éthyl]amino} pyrimidin-4-yl)acétonitrile, 1,3-benzothiazol-2-yl(2-{[2-(3-méthoxyphényl)éthyl]amino}pyrimidin-4-yl)acétonitrile, 1,3-benzothiazol-2-yl(2-{[2-(2-fluorophényl)éthyl] amino} pyrimidin-4-yl)acétonitrile, 1,3-benzothiazol-2-yl[2-({2-[3-(trifluorométhyl)phényl]éthyl} amino) pyrimidin-4yl]acétonitrile, 1,3-benzothiazol-2-yl{2-[(2-hydroxy-2-phényléthyl)amino]pyrimidin-4-yl} acétonitrile, 1,3-benzothiazol-2-yl{2- [(2-{[3-(trifluorométhyl) pyridin-2-yl]amino} éthyl)amino]pyrimidin-4-yl}acétonitrile, 1,3-benzothiazol-2-yl(2-{[2-(3-chlorophényl)éthyl]amino} pyrimidin-4-yl)-acétonitrile, 1,3-benzothiazol-2-yl(2-{[2-(3,4-dichlorophényl)éthyl]-amino} pyrimidin-4-yl)acétonitrile, 1,3-benzothiazol-2-yl(2-{[2-(4-méthoxyphényl) éthyl]amino} pyrimidin-4-yl)acétonitrile, 1,3-benzothiazol-2-yl(2-{[2-(4-méthylphényl)éthyl]amino}pyrimidin-4-yl) acétonitrile, 1,3-benzothiazol-2-yl(2-{[2-(3-fluorophényl)éthyl]amino} pyrimidin-4-yl)acétonitrile, 1,3-benzothiazol-2-yl(2-{[2-(4-phénoxyphényl)éthyl]amino}pyrimidin-4-yl)acétonitrile, 1,3-benzothiazol-2-yl -(2-{[2-(2-phénoxyphényl) éthyl] amino}pyrimidin-4-yl) acétonitrile, 1,3-benzothiazol-2-yl(2-{[2-( 4-bromophenyl)ethyl]amino} pyrimidin-4-yl)acétonitrile, 1,3 -benzothiazol-2-yl(2-{[2-( 4-fluorophényl)éthyl] -amino}pyrimidin-4-yl) acétonitrile, 1,3 -benzothiazol-2-yl {2-[(2-[1,1'-biphényl]-4-yléthyl)amino] pyrimidin-4-yl}acétonitrile, 1,3-benzothiazol-2-yl{2-[(2-{4-[hydroxy(oxido)amino]phényl} éthyl)amino]-pyrimidin-4yl} acétonitrile, 1,3-benzothiazol-2-yl(2-{[2-(1H-1,2,4-triazol-1-yl)éthyl]amino} pyrimidin-4-yl)acétonitrile, 1,3-benzothiazol-2-yl(2-{[3-(1H-pyrazol-1-yl) propyl]amino} pyrimidin-4-yl) acétonitrile, 4-[2-({4-[1,3-benzothiazol-2-yl(cyano)méthyl]pyrimidin-2-yl}amino) éthyl]benzènesulfonamide, {2-[(2-pyridin-3-yléthyl) amino]pyrimidin-4-yl}[5-(trifluorométhyl)-1,3-benzothiazol-2yl]acétonitrile, 1,3-benzothiazol-2-yl{2-[(1H- tétraazol-5-yl-méthyl)amino] pyrimidin-4-yl} acétonitrile, 1,3-benzothiazol-2-yl[2-(benzyloxy) pyrimidin-4-yl]acétonitrile, 1,3-benzothiazol-2-yl{2-((4-pyridin-3-yl benzyl)oxy]pyrimidin-4-yl} acétonitrile, 1,3-benzothiazol-2-yl[2-(pyridin-4-ylméthoxy)pyrimidin-4-yl]acétonitrile, 1,3-benzothiazol-2-yl[2-(pyridin-2- ylméthoxy)pyrimidin- 4-yl]acétonitrile, 1,3-benzo thiazol-2-yl[2-(3-pyridin-2-ylpropoxy) pyrimidin-4-yl]acétonitrile, 1,3-benzothiazol-2-yl{2-(4-méthoxybenzyl) oxy]pyrimidin-4-yl} acétonitrile, 1,3-benzothiazol-2-yl[2-(pyridin-3-ylméthoxy)pyrimidin-4-yl] acétonilrile, 1,3-benzothiazol-2-yl {2-[2-{4-méthoxyphényl)éthoxy]-pyrimidin-4-yl} acétonitrile, 1,3-benzothiazol-2-yl[2-([1,1'-biphényl]-3-yl- méthoxy)pyrimidin-4-yl]acétonitrile, 1,3-benzothiazol-2-yl 12-[(3,4,5-triméthoxybenzyl)oxylpyrimidin-4-yl} acétonitrile, 1,3-benzothiazol-2-yl{2-[(3,4-dichlorobenzy)oxy]pyrimidin-4-yl}acétonitrile, 1,3-benzothiazol-2-yl[2-({3-[(diméthylamino)méthyl]benzyl} oxy) pyrimidin-4-yl]acétonitrile, 1,3-benzothiazol-2-yl{2-[(1-oxidopyridin-3-yl)méthoxy]pyrimidin-4-yl} acétonitrile, 1,3-benzothiazol-2-yl(2-{[4-(morpholin-4- ylméthyl) benzyl]oxy} pyrimidin-4-yl)acétonitrile, 1,3-benzothiazol-2-yl {2-[(4-pyridin-2-yl)benzyl) oxy]pyrimidin-4-yl} acétonitrile, 1,3-benzothiazol-2-yl(2-{[4-(pipéridin-1-ylméthyl)benzyl]oxy} pyrimidin-4-yl)acétonitrile, 1,3-benzothiazol-2-yl[2- (4-méthoxyphénoxy) pyrimidin-4-yl] acétonitrile, 1,3-benzothiazol-2-yl[2-(4-butoxyphénoxy)pyrimidin-4-yl)acétonitrile, {2-[4-(4-acétylpipérazin-1-yl)phénoxy] pyrimidin-4-yl} (1,3-benzothiazol-2-yl)acétonitrile, [2-(4-méthoxyphénoxy)pyrimidin-4-yl] [5-(trifluorométhyl)-1,3-benzothiazol-2-yl]acétonitrile, N-[2-({4-[1,3-benzothiazol- 2-yl(cyano)méthyl]pyrimidin-2-yl}amino)éthyl]-4-chlorobenzamide, 1,3-benzothiazol-2-yl(2-méthoxy-4-pyrimidinyl) acétonitrile, 1,3-benzothiazol- 2-yl[2-({4-[(4- méthylpipérazin-1-yl) méthyl] benzyl} oxy)pyrimidin-4-yl]acétonitrile, 1,3-benzothiazol-2-yl[2-({4-[(4-benzyl-pipérazin-1-yl)méthyl]-benzyl) oxy)pyrimidin-4-yl]acétonitrile, 1,3-benzothiazol-2-yl(2-{(4-(pipérazin-1-ylméthyl)-benzyl]oxy} pyrimidin-4-yl)acétonitrile, 1,3-benzothiazol-2-yl[2-({4-[(4-formylpipérazin- l-yl)méthyl]benzyl) oxy)pyrimidin- 4-yl]acétonitrile, [2-({4-[(4-acétylpipérazin-1-yl)méthyl] benzyl}oxy)pyrimidin -4-yl](1,3-benzothiazol-2-yl)acétonitrile, (3H-benzothiazol-2-ylidène) -{2-[4-(4-[1,2,4]oxadiazol-3-ylméthyl-pipérazin-1-ylméthyl)benzyloxy] -pyrimidin-4-yl} acétonitrile, ester méthylique de l'acide 4-(4-{4-[(3H-benzothiazol-2-ylidène)- cyano- méthyl]- pyrimidin-2-yloxyméthyl}-benzyl)pipérazine-1-carboxylique, 2-[4-(4-{4-[(3H - benzothiazol-2-ylidène)-cyano-méthyl]-pyrimidin-2-yloxyméthyl}benzyl)-pipérazin-1-yl]-acétamide, (2-{4-[4-(2-amino-acétyl)- pipérazin -1-ylméthyl]-benzyloxy}-pyrimidin-4-yl)-(3H-benzothiazol-2-ylidène)-acétonitrile, ester méthylique de l'acide [4-(4-{4-[(3H-benzothiazol-2- ylidène) cyano-méthyl]- pyrimidin-2- yloxyméthyl) - benzyl) pipérazin-1-yl]-acétique, (3H -benzothiazol-2-ylidène)-(2-{4-[4-(2-méthoxy-éthyl)-pipérazin-1-ylméthyl]benzyloxy}-pyrimidin-4-yl)-acétonitrile, diméthylamide d'acide 4-(4-{4-[(3H-benzothiazol-2-ylidène)-cyano -méthyl]-pyrimidin-2-yloxyméthyl}-benzyl)pipérazine-1-carboxylique, (3H-benzothiazol-2-ylidène)-(2-{4-[4-éthyl- pipérazin-1- ylméthyl)benzyloxy}- pyrimidin-4-yl)-acétonitrile, (3H-benzothiazol-2-ylidène)-(2-{4-[4-(2-hydroxyéthyl)-pipérazin-1-ylméthyl]benzyloxy}-pyrimidin-4-yl)-acétonitrile.

9. Utilisation selon l'une quelconque des revendications précédentes, qui comprend en outre au moins un médicament supplémentaire choisi dans l'ensemble comprenant l'insuline, les inhibiteurs d'aldose réductase, les inhibiteurs d'α-glucosidase, les agents de sulfonylurée, les biguanides, les thiazolidines, les agonistes de PPAR, les inhibiteurs de GSK-3.

10. Utilisation selon la revendication 9, dans laquelle ledit médicament supplémentaire est choisi dans l'ensemble comprenant une insuline à action rapide, une insuline semi-lente, une insuline à action longue, une combinaison d'insuline semi-lente et à action rapide, Minalrestat, Tolrestat, Sorbinil, Methosorbinil, Zopolrestat, Epalrestat, Zenarestat, Imirestat, Ponalrestat, ONO-2235, GP-1447, CT-112, BAL-ARI 8, AD-5467, ZD5522, M-16209, NZ-314, M-79175, SPR-210, ADN 138 ou SNK-860, Miglitol, Acarbose, Glipizide, Glyburide, Chlorpropamide, Tolbutamide, Tolazamide ou Glimepriride.
